# EUROPÄISCHE PATENTSCHRIFT

(11) **EP 2 430 089 B1**
(45) Veröffentlichungstag und Bekanntmachung des Hinweises auf die Patenterteilung: **31.07.2013**
(21) Anmeldenummer: 10721438.9
(22) Anmeldetag: 11.05.2010
(51) Int. Cl.: C08K 5/3475, C07D 249/20

(54) **HOCHMOLEKULARE UNPOLARE BENZTRIAZOLE**
HIGH MOLECULAR WEIGHT NON-POLAR BENZOTRIAZOLES
BENZOTRIAZOLES À POIDS MOLÉCULAIRE ÉLÉVÉ

(30) Priorität: 15.05.2009 EP 09160348
(43) Veröffentlichungstag der Anmeldung: 21.03.2012
(73) Patentinhaber: BASF SE, 67056 Ludwigshafen (DE)
(72) Erfinder: HAREMZA, Sylke, 69151 Neckargemünd (DE); MISSKE, Andrea, 67346 Speyer (DE); SCHAMBONY, Simon, 67061 Ludwigshafen (DE); GLASER, Alban, 68199 Mannheim (DE)
(86) Internationale Anmeldenummer: PCT/EP2010/056489
(87) Internationale Veröffentlichungsnummer: WO 2010/130752

(56) Entgegenhaltungen:
- EP-A1- 0 057 160
- EP-A2- 0 718 341
- WO-A1-98/25922
- WO-A1-03/004490
- US-A1- 2007 095 432
- DATABASE WPI Week 198533 Thomson Scientific, London, GB; AN 1985-199544 XP002602568, -& JP 60 124635 A (ADEKA ARGUS CHEM CO LTD) 3. Juli 1985 (1985-07-03)

## Beschreibung

Die vorliegende Erfindung betrifft die Verwendung von bestimmten Benztriazolderivaten als UV-Absorber und zur Stabilisierung von unbelebten organischen Materialien. Weiterhin betrifft die Erfindung Verfahren zur Stabilisierung von unbelebten organischen Materialien, insbesondere Kunststoffen, gegen die Einwirkung von Licht unter Verwendung von spezifischen Benztriazolderivaten. Weitere Gegenstände der Erfindung sind bestimmte Benztriazolderivate und unbelebte organische Materialien, enthaltend bestimmte Benztriazolderivate.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt beziehungsweise ganz bevorzugt sind insbesondere auch diejenigen Ausführungsformen der vorliegenden Erfindung, in denen alle Merkmale des erfindungsgemäßen Gegenstandes die bevorzugten beziehungsweise ganz bevorzugten Bedeutungen haben.

Benztriazolderivate sind dem Fachmann allgemein als Lichtstabilisatoren oder UV-Absorber beispielsweise für Anwendungen in Kunststoffen, Lacken, Tinten oder in der Kosmetik aus dem Stand der Technik bekannt. Bei diesen Benztriazolderivaten handelt es sich jedoch häufig um niedermolekulare Verbindungen mit einem Molekulargewicht von deutlich unter 1000 g/mol.

EP 0 280 650 A1 beschreibt die Verwendung bestimmter Benzotriazolderivate als Lichtschutzmittel für Aufzeichnungsmaterialien für den Tintenstrahldruck. Die in dieser Schrift beschriebenen höhermolekularen Benzotriazole können mehrere Benzotriazolgruppen enthalten und erreichen damit Molekulargewichte, die 1000 g/mol überschreiten können. Diese höhermulekularen Benzotriazole zeichnen sich durch eine erhöhte Hydrophilie und Dispergierbarkeit in Öl-in-Wasser Emulsionen aus und werden daher entsprechend der Lehre der EP 0 280 650 A1 für wässrige Systeme und Tinten eingesetzt.

EP 0 057 160 A1 beschreibt 2-(2-Hydroxyphenyl)-benztriazole, ihre Verwendung als UV-Absorber und ihre Herstellung. Die UV-Absorber werden insbesondere zur Stabilisierung von Lacken und Photomaterial verwendet. Es werden nur Derivate mit einer oder zwei Benztriazolgruppen und einer Molekülmasse von in der Regel weniger als 1000 g/mol beschrieben.

In der US 3,213,058 werden o-Hydroxyphenylbenztriazole, umfassend eine Benztriazolgruppe, deren Verwendung als UV-Absorber in Kunststoffen und Kunststoffe enthaltend diese o-Hydroxyphenylbenztriazole beschrieben. Weiterhin beschreibt die US 3,213,058 mit diesen o-Hydroxyphenylbenztriazole modifizierte Polymere, nämlich Harze basierend auf Estern der o-Hydroxyphenylbenztriazole mit hydroxylhaltigen oder carboxylhaltigen copolymerisierten Monomeren.

Lichtstabilisatoren und UV-Absorber auf Basis der oben genannten niedermolekularen Benztriazolderivate sind daher aus dem Stand der Technik bekannt.

Häufig tritt bei der Ausrüstung von unbelebten organischen Materialien, insbesondere Kunststoffen (Polymeren), mit UV-Absorbern oder Stabilisatoren das Problem auf, dass die UV-Absorber oder Stabilisatoren im Laufe der Zeit die Polymermatrix verlassen, beispielsweise indem sie an die Oberfläche des Polymeren wandern (Migration). Dies ist insbesondere kritisch, falls durch die Migration UV-Absorber oder Stabilisatoren in Kontakt mit Lebensmitteln gelangen, indem sie beispielsweise aus einem Verpackungsmaterial in den verpackten Stoff gelangen.

Weiterhin existiert daher das Bedürfnis die Stabilität (Lebensdauer) von UV-Absorbern oder Stabilisatoren in unbelebten organischen Materialien zu erhöhen.

Unbefriedigend ist oft auch die mangelhafte Verträglichkeit der UV-Absorber oder Stabilisatoren mit unbelebten organischen Materialien, beispielsweise Polyolefinen oder anderen Kunststoffen, und die thermische Zersetzung der Stabilisatoren beim Einarbeiten in Polymere bei erhöhter Temperatur.

Der Erfindung lag daher die Aufgabe zugrunde UV-Absorber oder Stabilisatoren zur Verfügung zu stellen, welche zu einer weiteren Unterdrückung der Migration aus unbelebten organischen Materialien führen. Weiterhin sollten diese UV-Absorber oder Stabilisatoren Verbesserungen in der Dauer der Stabilisierung von unbelebtem organischem Material mit sich bringen. Eine weitere Teilaufgabe der Erfindung war es UV-Absorber oder Stabilisatoren zur Verfügung zu stellen, die eine gute Verträglichkeit mit den zu stabilisierenden Materialien, etwa Polyolefinen und anderen Kunststoffen und Beständigkeit bei der Einarbeitung in das zu stabilisierende Material, aufweisen.

Diese und andere Aufgaben werden, wie aus dem Offenbarungsgehalt der vorliegenden Erfindung ersichtlich, durch die verschiedenen Ausführungsformen der erfindungsgemäßen Verwendung von Benztriazolderivaten der allgemeinen Formel (I) 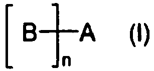 wobei
- B: eine gegebenenfalls substituierte 2-(2-Hydroxyphenyl)-2H-benztriazolgruppe,
- n: eine ganze Zahl aus dem Bereich von 3 bis 20 und
- A: ein n-valentes organisches Radikal
ist,
als UV-Absorber oder Stabilisator in unbelebten organischen Materialien,
wobei A einem n-valenten organischen Radikal der allgemeinen Formeln (III), (IIIa) oder (IIIb) oder einem tri-valenten organischen Radikal der allgemeinen Formel (IIId) entspricht 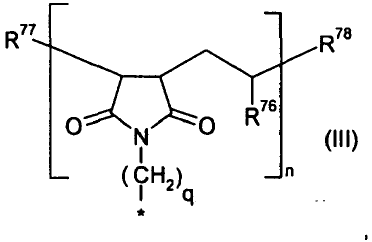 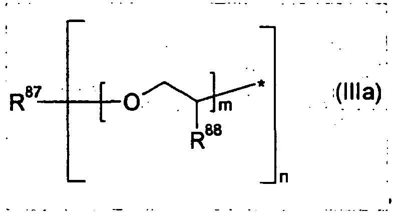 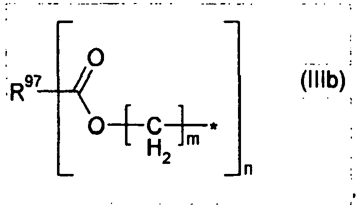 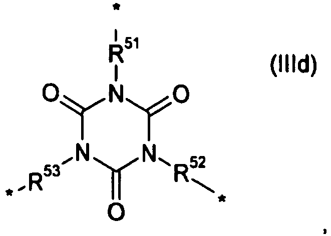 mit
- m: unabhängig voneinander eine ganze Zahl aus dem Bereich 0 bis 10,
- q: ganze Zahl aus dem Bereich von 0 bis 10,
- R⁵¹, R⁵², R³³: unabhängig voneinander, C₁-C₂₀-Alkylen,
- R⁷⁶: H, Aryl, C₁-C₃₀-Alkyl,
- R⁷⁷: H, C₁-C₂₀-Alkyl, C₁-C₂₀-Alkoxy,
- R⁷⁸: H, C₁-C₂₀-Alkyl, C₁-C₂₀-Alkoxy,
- R⁸⁷: lineares oder verzweigtes C₁-C₂₀-Alkan, bei dem n Wasserstoffe durch eine Bindung ersetzt sind,
- R⁸⁸: H, linear oder verzweigtes C₁-C₂₀-Alkyl,
- R⁹⁷: lineares oder verzweigtes C₁-C₂₀-Alkan, bei dem n Wasserstoffe durch eine Bindung ersetzt sind,
und
wobei * die Anbindung des Benztriazolderivats an das n-valente organische Radikal der Formeln (III), (IIIa) oder (IIIb) oder an das tri-valente organische Radikal (IIId) bezeichnet, gelöst.

Selbstverständlich sind auch Gemische von Benztriazolderivaten der allgemeinen Formel (I) als UV-Absorber oder Stabilisator in unbelebten organischen Materialien geeigent.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Halogen steht für Fluor, Chlor, Brom, oder Iod, vorzugsweise für Fluor, Chlor oder Brom, besonders bevorzugt für Chlor.

Im Einzelnen haben die für die verschiedenen Substituenten angegebenen Sammelbegriffe folgende Bedeutung:
C₁-C₂₀-Alkyl: geradkettige oder verzweigte Kohlenwasserstoffreste mit bis zu 20 Kohlenstoffatomen, beispielsweise C₁-C₁₀-Alkyl oder C₁₁-C₂₀-Alkyl, bevorzugt C,-C,o-Alkyl beispielsweise C₁-C₃-Alkyl, wie methyl, Ethyl, Propyl, Isopropyl, oder C₄-C₆-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Tri-methylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethybutyl, Nonyl oder Decyl sowie deren Isomere.

Aryl: ein ein- bis dreikerniges aromatisches Ringsystem enthaltend 6 bis 14 Kohlenstoffringglieder, z. B. Phenyl, Naphthyl oder Anthracenyl, bevorzugt ein ein- bis zweikerniges, besonders bevorzugt ein einkerniges aromatisches Ringsystem.

Arylalkyl ist ein ein- bis dreikerniges aromatisches Ringsystem (wie vorstehend genannt), welches über eine C₁-C₂₀-Alkylengruppe, bevorzugt eine C₁-C₁₄-Alkylengruppe, angebunden ist, bevorzugt ein ein- bis zweikerniges, besonders bevorzugt ein einkerniges aromatisches Ringsystem.

C₁-C₂₀-Alkylen: geradkettige oder verzweigte Kohlenwasserstoffreste mit 1 bis 20 Kohlenstoffatomen, beispielsweise C₁-C₁₀-Alkylen oder C₁₁-C₂₀-Alkylen, bevorzugt C₁-C₁₀-Alkylen, insbesondere Methylen, Dimethylen, Trimethylen, Tetramethylen, Pentamethylen oder Hexamethylen.

C₁-C₂₀-Alkoxy bedeutet eine geradkettige oder verzweigte Alkylgruppe mit 1 bis 20 Kohlenstoffatomen (wie vorstehend genannt), welche über ein Sauerstoffatom (-O-) an gebunden sind, beispielsweise C₁-C₁₀-Alkoxy oder C₁₁-C₂₀-Alkoxy, bevorzugt C₁-C₁₀-Alkyloxy, insbesondere bevorzugt C₁-C₃-Alkoxy, wie beispielweise Methoxy, Ethoxy, Propoxy.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl sowie ein gesättigtes oder ungesättigtes mehrcyclisches System wie z. B. Norbornyl oder Norbenyl. Besonders bevorzugt C₅-C₆-Cycloalkyl.

Heteroatome sind Phosphor, Sauerstoff, Stickstoff oder Schwefel bevorzugt Sauerstoff, Stickstoff oder Schwefel, deren freie Valenzen gegebenenfalls durch H-Atome abgesättigt sind.

UV-Absorber absorbieren UV-Licht mit einer Wellenlänge kleiner 400 nm, insbesondere von 200 bis 400 nm. UV-Absorber können daher beispielsweise UV-A (von 320 bis 400 nm), UV-B (von 290 bis 319 nm) und/oder UV-C (von 200 bis 289 nm) Licht absorbieren. Bevorzugt absorbieren UV-Absorber UV-A und/oder UV-B Licht. Ganz besonders bevorzugt absorbieren UV-Absorber UV-A und/oder UV-B Licht und desaktiviert die aufgenommene Lichtenergie strahlungslos.

In einer bevorzugten Ausführungsform der Erfindung werden als Benztriazolderivate solche der allgemeinen Formel (la) 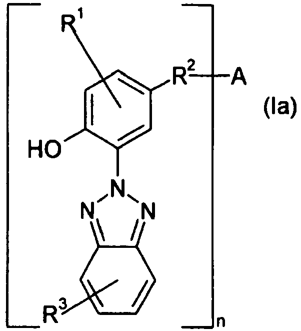 mit
- n: eine ganze Zahl aus dem Bereich von 3 bis 20,
- R¹: H, C₁-C₂₀-Alkyl, Arylalkyl, C₅-C₂₀-Alkoxycarbonylalkyl, C₃-C₁₅-Cycloalkyl,
- R³: H, Halogen, CF₃, COOR⁴,
- R⁴: H, C₁-C₂₀-Alkyl,
- R²: eine Einfachbindung, O, NR⁷ oder eine Gruppe der allgemeinen Formel (IIa) 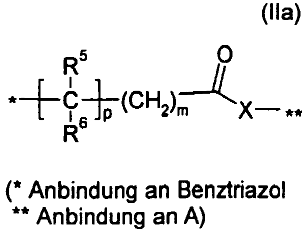
- m: eine ganze Zahl aus dem Bereich von 0 bis 4,
- p: 0 oder 1,
- A: ein n-valentes organisches Radikal darstellt,
- R⁵: H, C₁-C₂₀-Alkyl,
- R⁶: H, C₁-C₂₀-Alkyl,
- X: O, NR⁷,
- R⁷: H, C₁-C₂₀-Alkyl,
als UV-Absorber oder Stabilisatoren in unbelebten organischen Materialien eingesetzt. Ganz bevorzugt ist hierbei in Formel (la) n eine ganze Zahl aus dem Bereich von 3 bis 10, insbesondere von 3 bis 6. Ganz bevorzugt ist R³ hierbei H oder Cl, insbesondere ist R³ an der 3-Position der Benztriazolgruppe fixiert. Ganz bevorzugt ist hierbei in Formel (IIa) m eine ganze Zahl aus dem Bereich von 1 bis 3, besonders bevorzugt 1 oder 2, insbesondere 2. Ganz bevorzugt ist hierbei p=0, ebenfalls ganz bevorzugt ist hierbei p=1. Bevorzugt ist R⁷ H.

In einer weiteren bevorzugten Ausführungsform der Erfindung werden als Benztriazolderivate solche der allgemeinen Formel (Ib) 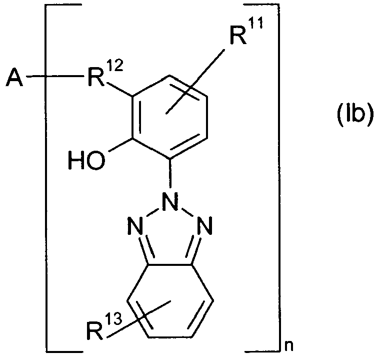 mit
- R¹¹: H, C₁-C₂₀-Alkyl, Arylalkyl, C₅-C₂₀-Alkoxycarbonylalkyl, C₃-C₁₅-Cycloalkyl,
- R¹³: H, Halogen, CF₃, COOR¹⁴,
- R¹⁴: H, C₁-C₂₀-Alkyl,
- R¹²: eine Einfachbindung, O, NR⁷ oder eine Gruppe der allgemeinen Formel (IIa)
als UV-Absorber oder Stabilisatoren in unbelebten organischen Materialien eingesetzt, wobei die restlichen Substituenten und Indizes die oben für (la) angegebenen Bedeutungen haben. Ganz bevorzugt ist hierbei in Formel (Ib) n eine ganze Zahl aus dem Bereich von 3 bis 10, insbesondere von 3 bis 6. Ganz bevorzugt ist R¹³ hierbei H oder Cl, insbesondere ist R¹³ an der 3-Position der Benztriazolgruppe fixiert. Ganz bevorzugt ist hierbei in Formel (IIa) m eine ganze Zahl aus dem Bereich von 1 bis 3, besonders bevorzugt 1 oder 2, insbesondere 2. Ganz bevorzugt ist hierbei p=0, ebenfalls ganz bevorzugt ist hierbei p=1.

In einer bevorzugten Ausführungsform der Erfindung werden als Benztriazolderivate solche der allgemeinen Formel (Ic) 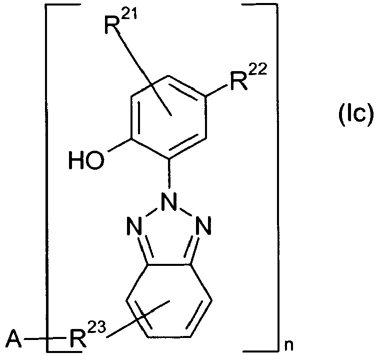 mit
- R²¹: H, C₁-C₂₀-Alkyl, Arylalkyl, C₅-C₂₀-Alkoxycarbonylalkyl, C₃-C₁₅-Cycloalkyl,
- R²²: H, C₁-C₂₀-Alkyl, COOR²⁴,
- R²⁴: H, C₁-C₂₀-Alkyl,
- R²³: eine Einfachbindung, O, NR⁷ oder eine Gruppe der allgemeinen Formel (IIa)
als UV-Absorber oder Stabilisatoren in unbelebten organischen Materialien eingesetzt, wobei die restlichen Substituenten und Indizes die oben für (Ia) angegebenen Bedeutungen haben. Ganz bevorzugt ist hierbei in Formel (Ic) n eine ganze Zahl aus dem Bereich von 3 bis 10, insbesondere von 3 bis 6. Ganz bevorzugt ist R²² hierbei C₁-C₂₀-Alkyl. Ganz bevorzugt ist hierbei in Formel (IIa) m eine ganze Zahl aus dem Bereich von 1 bis 3, besonders bevorzugt 1 oder 2, insbesondere 2. Ganz bevorzugt ist hierbei p=0, ebenfalls ganz bevorzugt ist hierbei p=1.

Verfahren zur Herstellung von Benztriazolderivaten sind dem Fachmann bekannt. Der Benztriazol-Ring lässt sich in der Regel durch eine Folge von Diazotierung, Kupplung und Reduktion aus den entsprechenden Aminophenyl und Phenol herstellen, wie zum Beispiel in EP 0214102 beschrieben. Die weitere Derivatisierung der Benztriazole oder/und Phenol-Ringe ist zum Beispiel in EP 57160 beschrieben.

Bevorzugt beträgt das Molekulargewicht (Zahlenmittel Mn) der Benztriazolderivate der allgemeinen Formel (I), (Ia), (Ib) oder (Ic) von 1100 g/mol bis 5000 g/mol, besonders bevorzugt von 1500 bis 5000 g/mol, insbesondere von 2000 bis 5000 g/mol.

Die n-valenten organischen Radikale A beruhen in der Regel auf organischen Verbindungen, die mit einer Anzahl n von Benztriazolderivaten, enthaltend beispielsweise die Benzotriazolsubstituenten der Formeln (la) - (Ic), umgesetzt werden. Beispielsweise sind die n-valenten organischen Radikale lineare oder verzweigte Kohlenwasserstoffreste, die gegebenenfalls jeweils an beliebiger Position durch ein oder mehrere, Heteroatome unterbrochen sein können. Beispielsweise kommen als organische Verbindungen auf denen die n-valenten organischen Radikale beruhen Polyole, Polyesterpolyole, Polyetherpolyole, Polyetheramine und Epoxidverbindungen, Polymere enthaltend Maleinsäuregruppen oder Derivate von Maleinsäuregruppen, Polycarbonsäuren, Cyanursäure oder Cyanurchloridderivate, insbesondere ethoxylierte oder propoxylierte Polyole in Frage. Bevorzugt sind hierbei Polymere enthaltend Maleinsäuregruppen oder Derivate von Maleinsäuregruppen, Polyole, insbesondere ethoxylierte oder propoxylierte Polyole oder Polyetheramine.

Geeignete Polyetheramine umfassen beispielsweise Tri-aminopolyalkylenoxid-Verbindungen. Darunter ist zu verstehen, dass diese Verbindungen einerseits drei A-mino-Funktionen (NH-bzw. NH₂-Funktionen), andererseits Alkylenoxid-Bausteine enthalten. Bei den letztgenannten Bausteinen handelt es sich insbesondere um Ethylenoxid und/oder Propylenoxid und/oder Butylenoxid.

Die Polyetheramine können einzeln oder in Form von Mischungen verwendet werden.

In einer weiteren Ausführungsform werden Umsetzungsprodukte der Polyole mit Propylenoxid und/oder Ethylenoxid eingesetzt, wobei pro Mol Polyol bevorzugt 1 bis 5 mol Propylen- und/oder Ethylenoxid eingesetzt werden.

In einer bevorzugten Ausführungsform der Erfindung werden solche Benztriazolderivate verwendet, bei denen die Gruppe A einem n-valenten organischen Radikal der allgemeinen Formeln (III) entspricht 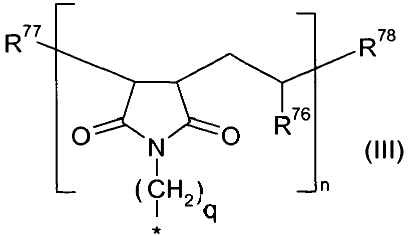 mit
- n: eine ganzen Zahl aus dem Bereich von 3 bis 20,
- q: ganze Zahl aus dem Bereich von 0 bis 10,
- R⁷⁶: H, Aryl, C₁-C₃₀-Alkyl,
- R⁷⁷: H, C₁-C₂₀-Alkyl, C₁-C₂₀-Alkoxy,
- R⁷⁸: H, C₁-C₂₀-Alkyl, C₁-C₂₀-Alkoxy,
wobei * die Anbindung des Benztriazolderivats an das n-valente organischen Radikal der Formel (III) bezeichnet.

Ganz bevorzugt ist hierbei in Formel (III) n eine ganze Zahl aus dem Bereich von 3 bis 10, insbesondere von 3 bis 6. Ganz bevorzugt ist hierbei q eine ganze Zahl aus dem Bereich von 2 bis 10, besonders bevorzugt aus dem Bereich von 2 bis 4, insbesondere bevorzugt 2 oder 3, insbesondere 2. Ganz bevorzugt ist R⁷⁶ hierbei H, Aryl, C₁-C₃₀-Alkyl, bevorzugt C₂-C₂₀-Alkyl, besonders bevorzugt C₁₀-C₂₀-Alkyl, insbesondere C₁₈-Alkyl oder C₂₀-Alkyl.

In einer weiteren bevorzugten Ausführungsform der Erfindung werden solche Benztriazolderivate verwendet, bei denen die Gruppe A einem n-valenten organischen Radikal der allgemeinen Formeln (IIIa) entspricht 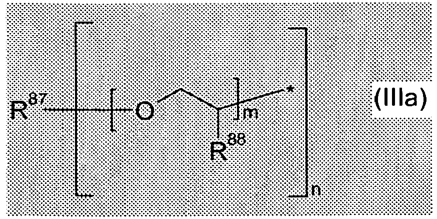 mit
- n: eine ganzen Zahl aus dem Bereich von 3 bis 20,
- m: unabhängig voneinander eine ganzen Zahl aus dem Bereich 0 bis 10,
- R⁸⁷: lineares oder verzweigtes C₁-C₂₀-Alkan, bei dem n Wasserstoffe durch eine Bindung ersetzt sind,
- R⁸⁸: H, linear oder verzweigt C₁-C₂₀-Alkyl,
wobei * die Anbindung der Benztriazolderivate an das n-valente organischen Radikal der Formel (IIIa) bezeichnet. Ganz bevorzugt ist hierbei in Formel (IIIa) n eine ganze Zahl aus dem Bereich von 3 bis 10, insbesondere von 3 bis 6. Ganz bevorzugt ist m eine ganze Zahl von 0 bis 5, insbesondere von 0 bis 3. Ganz bevorzugt ist R⁸⁷ ein C₁-C₁₀-Alkan, insbesondere ein C₁-C₅-Alkan bei dem n Wasserstoffe durch eine Bindung ersetzt sind. Ganz bevorzugt ist R⁸⁸ hierbei H oder Methyl.

In einer weiteren bevorzugten Ausführungsform der Erfindung werden solche Benztriazolderivate verwendet, bei denen die Gruppe A einem n-valenten organischen Radikal der allgemeinen Formeln (IIIb) entspricht 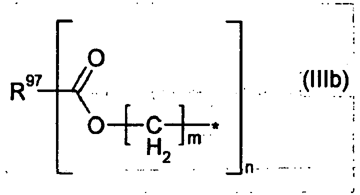 mit
- n: eine ganzen Zahl aus dem Bereich von 3 bis 20,
- m: unabhängig voneinander eine ganzen Zahl aus dem Bereich 0 bis 10,
- R⁹⁷: lineares oder verzweigtes C₁-C₂₀-Alkan, bei dem n Wasserstoffe durch eine Bindung ersetzt sind
wobei * die Anbindung der Benztriazolderivate an das n-valente organischen Radikal der Formel (IIIb) bezeichnet. Ganz bevorzugt ist hierbei in Formel (IIIb) n eine ganze Zahl aus dem Bereich von 3 bis 10, insbesondere von 3 bis 6. Ganz bevorzugt ist m eine ganze Zahl von 0 bis 5, insbesondere von 0 bis 3. Ganz bevorzugt ist R⁹⁷ ein C₁-C₁₀-Alkan, insbesondere ein C₁-C₅-Alkan bei dem n Wasserstoffe durch eine Bindung ersetzt sind.

In einer weiteren bevorzugten Ausführungsform der Erfindung werden solche Benztriazolderivate verwendet, bei denen die Gruppe A einem tri-valenten organischen Radikal der allgemeinen Formeln (IIId) entspricht 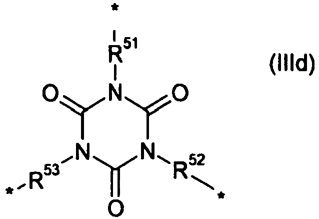 mit
- R⁵¹, R⁵², R³³: unabhängig voneinander, C₁-C₂₀-Alkylen,
wobei * die Anbindung der Benztriazolderivate an das tri-vafente organischen Radikal der Formel (IIId) bezeichnet. Ganz bevorzugt sind R⁵¹, R⁵², R⁵³ unabhängig voneinander C₁-C₅-Alkylen, insbesondere C₂-C₃-Alkylen.

In einer weiteren Ausführungsform der Erfindung erfolgt die Anknüpfung des n-wertigen organischen Radikals A an die oben genannten Benztriazolderivate der allgemeinen Formel (la), (Ib) oder (Ic) durch die Reaktion eines mindestens n-wertigen Alkohols A' mit einer Verbindung der allgemeinen Formel (IV), (IVa) bzw. (V) 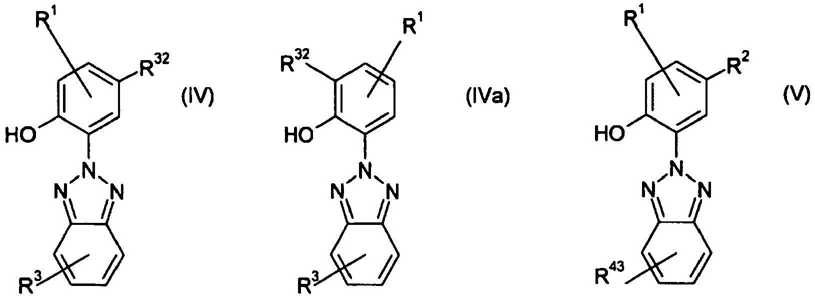 mit
- R³², R⁴³: eine Einfachbindung oder eine Gruppe der allgemeinen Formel (VI) 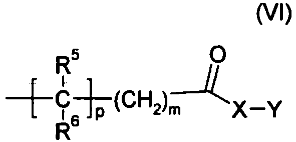
- m: eine ganzen Zahl aus dem Bereich von 0 bis 4,
- p: 0 oder 1,
- X: O, NR⁷,
- R⁷: H, C₁-C₂₀-Alkyl, C₁-C₂₀-Alkoxy,
- Y: H, C₁-C₂₀-Alkyl, C₁-C₂₀-Alkoxy,
unter der Maßgabe, dass mindestens drei Benztriazolderivate der allgemeinen Formel (IV), (IVa) oder (V) mit jeweils einem n-wertigen Alkohol A' umgesetzt werden. Die übrigen Substituenten haben hierbei die oben für die Formeln (la), (Ib) und (Ic) angegebenen Bedeutungen. Ganz bevorzugt bezeichnet m in Formel (VI) eine ganzen Zahl aus dem Bereich von 0 bis 4, besonders bevorzugt von 1 bis 3, ganz besonders bevorzugt 1 oder 2, insbesondere 2. Ganz bevorzugt ist p hierbei 0 oder 1, besonders bevorzugt 0 und ebenfalls besonders bevorzugt 1.

Bevorzugt wird als n-wertiger Alkohol A' ein Triol, Tetrol, Pentol, Hexol oder Polyol eingesetzt. Ganz bevorzugt sind als Triole Trimethylolpropan, Glycerin. AlsTetrol ist Pentaerythrit, als Pentol Sorbit und als Hexol Mannitol bevorzugt.

In einer bevorzugten Ausführungsform der erfindungsgemäßen Verwendung werden die oben aufgezählten Benztriazolderivate als UV-Absorber in Kunststoffen oder Wachsen verwendet, wobei die Kunststoffe beispielsweise Polyolefine, wie Polyethylen (PE) oder Polypropylen (PP), Polycarbonate, Polyester, wie Polyethylenterephtalat (PET), Polyvinylchlorid (PVC), Polystyrol (PS), Polystyrolcopolymere, wie schlagfest modifiziertes PS (HIPS), Styrol-Acrylnitril-Copolymer (SAN), Acryinitril-Styrol-Acrylester-Copolymer (ASA), oder Acrylnitril-Butadien-Styrol-Copolymer (ABS), oder auch Poliolefinwachse enthalten. Kunstoffe können auch Copolymere oder Mischungen (Blends) der genannten Polymere enthalten oder sein. Bevorzugt Kunstoffe sind Polyolefine und Polycarbonate.

Die Einarbeitung der Benztriazolderivate in unbelebte organische Materialien, insbesondere in Kunststoffe erfolgt im Allgemeinen durch das Mischen der Bestandteile. Beispielsweise erfolgt das Mischen durch dem Fachmann bekannte Verfahren wie sie bei der Additivierung von Kunststoffen allgemein eingesetzt werden. Die Benztriazolderivate in fester, flüssiger oder gelöster Form werden bevorzugt zur Ausrüstung von Kunststoffen eingesetzt. Die Benztriazolderivate lassen sich zu diesem Zweck sowohl als feste oder flüssige Formulierung, als auch als Pulver nach den üblichen Methoden in die Kunststoffe einarbeiten. Zu nennen ist hier beispielsweise die Mischung der Teilchen mit den organischen Polymeren vor oder während eines Extrusionsschrittes. Weitere Beispiele für die Ausrüstung oder Stabilisierung von Kunststoffen mit Additiven können dem Plastics Additives Handbook, 5. Auflage, Hanser Verlag, ISBN 1-56990-295-X entnommen werden. Die additivierten Kunststoffe können beispielsweise als Granulate, Pellets, Pulver, Folien oder Fasern vorliegen.

In einer weiteren bevorzugten Ausführungsform der erfindungsgemäßen Verwendung werden die oben aufgezählten Benztriazolderivate als UV-Absorber in Kunststoff-Formkörpern eingesetzt. Kunststoff-Formkörper sind bevorzugt Agrarfolien, Spritzgußteile, die farbig sein können, PC-Platten, PET-Fasern, PET-Verpackungen, flammgeschützte PP-Fasern, Verpackungsfolien, insbesondere aus PE, Lebensmittelverpackungen, Kerzen. Besonders bevorzugt sind Verpackungsfolien, insbesondere aus PE.

Die Herstellung von Kunststoff-Formkörpern erfolgt durch dem Fachmann bekannte Verfahren. Insbesondere lassen sich die Kunststoff-Formkörper durch Extrusion oder Coextrusion, Compoundierung, Verarbeitung von Granulaten oder Pellets, Spritzguss, Blasformung oder Knetung herstellen. Bevorzugt erfolgt die Verarbeitung durch Extrusion oder Coextrusion zu Folien (vgl. Saechtling Kunststoff Taschenbuch, 28. Auflage, Karl Oberbach, 2001).

Die unbelebten organischen Materialien, insbesondere die Kunststoffe oder Kunstoff-Formkörper enthalten von 0,01 bis 10 Gew.-% der oben aufgezählten Benzotriazole bezogen auf die Gesamtmenge an unbelebtem organischen Material oder Kunststoff. In einer Ausführungsform werden bevorzugt von 0,05 bis 1 Gew.-% Benztriazolderivate dem zugesetzt. In einer anderen Ausführungsform werden bevorzugt von 5 bis 8 Gew.-% Benztriazolderivate zugesetzt.

In einer speziellen Ausführung enthalten die unbelebten organischen Materialien, insbesondere die Kunststoffe oder Kunstoff-Formkörper eine thermoplastische Polymerkomponente oder bestehen aus einer thermoplastischen Polymerkomponente. Thermoplaste zeichnen sich durch ihre gute Verarbeitbarkeit aus und können im erweichten Zustand z. B. durch Pressen, Extrudieren, Spritzgießen oder andere Formgebungsverfahren zu Formteilen verarbeitet werden.

Die unbelebten organischen Materialien, insbesondere die Kunststoffe oder Kunstoff-Formkörper können zusätzlich wenigstens ein weiteres Additiv enthalten, dass vorzugsweise ausgewählt ist unter Farbmitteln, Antioxidantien, Stabilisatoren, z.B. Hindered Amine Light Stabilisatoren (HALS),anderen UV-Absorbern, Nickelquenchern, Metalldesaktivatoren, Verstärkungs- und Füllstoffen, Beschlagverhinderungsmittel (Antifog), Bioziden, Säurefängern, Antistatika, IR-Absorbern für langwellige IR Strahlung, Antiblockmittel wie SiO2, Lichtstreuern wie MgO oder TiO2, anorganischen oder organischen Reflektoren (beispielsweise Aluminium-Flakes).

Die Gesamtmenge an optionalen weiteren Additiven in der Zusammensetzung beträgt bis zu 15 Gew.-% bezogen auf die Gesamtmenge an unbelebtem organischen Material oder Kunststoff. Bevorzugt beträgt die Menge dieser Additive 0,5 bis 15 Gew.-%, ganz bevorzugt 0,5 bis 10 Gew.-% und insbesondere 0,5 bis 7,5 Gew.-%.

Als optionale weitere Stabilisatoren sind hierbei beispielsweise Phosphite, Phosphonite, Phosphine, Gehinderte Amine (HALS-Verbindungen), Hydroxylamine, Phenole, mit Acryloyl modifizierte Phenole, Peroxid-Zersetzer, Benzofuranonderivative oder Mischungen aus diesen zu nennen. Häufig sind Stabilisatoren kommerziell erhältlich, beispielsweise unter folgenden Handelsnamen IRGAPHOS® 168, DOVERPHOS® S-9228, ULTRANOX® 641 der Firmen Ciba und Dover. Weiterhin können zusätzlich zu den Stabilisatoren auch Costabilisatoren eingesetzt werden um die thermische Stabilität zu erhöhen.

Bevorzugte optionale weitere Stabilisatoren als weitere Additive sind Phosphite oder HALS-Verbindungen. Ganz bevorzugt sind HALS-Verbindungen der Firma Ciba, die unter dem Handelsnamen Chimassorb®, insbesondere Chimasorb® 119 FL, 2020, 940, oder Tinuvin®, insbesondere Tinuvin® 111, 123, 492, 494, 622, 765, 770, 783, 791, C 353, erhältlich sind. Ebenfalls ganz bevorzugt sind HALS-Verbindungen der Firma BASF SE die unter dem Handelsnamen Uvinul® erhältlich sind, insbesondere Uvinul® 4050 H (CAS-Nr. 124172-53-8), Uvinul® 4077 H (CAS-Nr. 52829-07-9) oder Uvinul® 5050 H (CAS-Nr. 152261-33-1).

Im Allgemeinen werden die optionalen Stabilisatoren als weitere Additive in einer Menge von 0,001 bis 3 Gew.-% bezogen auf bezogen auf die Gesamtmenge an unbelebtem organischen Material oder Kunststoff eingesetzt, bevorzugt von 0,002 bis 2 Gew.-%, ganz bevorzugt von 0,003 bis 1 Gew.-% und insbesondere von 0,005 bis 0,5 Gew.-%.

Als optionale weitere UV-Absorber kommen beispielsweise die kommerziell erhältlichen Verbindungen der Tinuvin®, insbesondere Tinuvin® 234, 326, 327, 328 oder Uvinul® Produktfamilien der Firmen Ciba beziehungsweise BASF SE in Frage.

Die Uvinul® Lichtschutzmittel umfassen Verbindungen der folgenden Klassen: Benzophenone, Benzotriazole, Cyanoacrylate, Zimtsäureester, para-Aminobenzoate, Naphthalimide. Darüber hinaus werden weitere bekannte Chromophore eingesetzt, z.B. Hydroxyphenyltriazine oder Oxalanilide.

Weitere Beispiele für optionale weitere UV-Absorber sind:
substituierte Acrylate, wie z.B. Ethyl- oder Isooctyl-α-cyano-β,β-diphenylacrylat (hauptsächlich 2-Ethylhexyl-α-cyano-β,β-diphenylacrylat), Methyl-α-methoxycarbonyl-β-phenylacrylat, Methyl-α-methoxycarbonyl-β-(p-methoxyphenyl)acrylat, Methyl- oder Butyl-α-cyano-β-methyl-β-(p-methoxyphenyl)acrylat, N-(β-methoxycarbonyl-β-cyanovinyl)-2-methylindolin, Octyl-p-methoxycinnamat, Isopentyl-4-methoxycinnamat, Urocaninsäure oder deren Salze oder Ester;

Derivate der p-Aminobenzoesäure, insbesondere deren Ester z.B. 4-Aminobenzoesäure-ethylester oder ethoxylierte 4-Aminobenzoesäureethylester, Salicylate, substituierte Zimtsäureester (Cinnamate) wie Octyl-p-methoxycinnamat oder 4-Isopentyl-4-methoxycinnamat, 2-Phenylbenzimidazol-5-sulfonsäure oder ihre Salze.

2-Hydroxybenzophenonderivative, wie z.B. 4-Hydroxy-, 4-Methoxy-, 4-Octyloxy-, 4-Decyloxy-, 4-Dodecyloxy-, 4-Benzyloxy-, 4,2',4'- Trihydroxy-, 2'-Hydroxy-4,4'-dimethoxy-2-hydroxybenzophenon sowie 4-Methoxy-2-hydroxybenzophenon-Sulfonsäure-Natriumsalz;
Ester der 4,4-Diphenylbutadien-1,1-dicarbonsäure, wie z.B. der Bis(2-ethylhexyl)ester;
2-Phenylbenzimidazol-4-sulfonsäure sowie 2-Phenylbenzimidazol- 5-sulfonsäure oder deren Salze;
Derivate von Benzoxazolen;
Derivate von Benztriazolen oder 2-(2'-Hydroxyphenyl)benztriazolen, wie z.B. 2-(2H-Benztriazol-2-yl)-4-methyl-6-(2-methyl-3- ((1,1,3,3-tetramethyl-1-(trimethylsilyloxy)disiloxanyl)-propyl)- phenol, 2-(2'-Hydroxy-5'-methylphenyl)benztriazol, 2-(3',5'-Di-tert.-butyl-2'-hydroxyphenyl)benztriazol, 2-(5'-tert.-Butyl-2'- hydroxyphenyl)benztriazol, 2-[2'-Hydroxy-5'-(1,1,3,3-tetramethylbutyl)phenyl]benztriazol, 2-(3',5'-Di-tert.-butyl-2'-hydroxyphenyl)-5-chlorbenztriazol, 2-(3'-tert.-Butyl-2'-hydroxy-5'-methylphenyl)-5-chlorbenztriazol, 2-(3'-sec.-Butyl-5'-tert.-butyl- 2'-hydroxyphenyl)benztriazol, 2-(2'-Hydroxy-4'-octyloxyphenyl)-benztriazol, 2-(3',5'-Di-tert.-amyl-2'-hydroxyphenyl)benztriazol, 2-[3',5'-Bis(α,α-dimethylbenzyl)-2'-hydroxyphenyl]benztriazol, 2-[3'-tert.-Butyl-2'-hydroxy-5'-(2-octyloxycarbonylethyl)phenyl]- 5-chlorbenztriazol, 2-[3'-tert.-Butyl-5'-(2-(2-ethylhexyloxy)- carbonylethyl)-2'-hydroxyphenyl]-5-chlorbenztriazol, 2[3'-tert.-Butyl-2'-hydroxy-5'-(2-methoxycarbonylethyl)phenyl]-5-chlorbenztriazol, 2-[3'-tert.-Butyl-2'-hydroxy-5'-(2-methoxycarbonylethyl)phenyl]benztriazol, 2-[3'-tert.-Butyl-2'-hydroxy-5'-(2-octyloxycarbonylethyl)phenyl]benztriazol, 2-[3'-tert.-Butyl-5'-(2- (2-ethylhexyloxy)carbonylethyl)-2'-hydroxyphenyl]benztriazol, 2-(3'-Dodecyl-2'-hydroxy-5'-methylphenyl)benztriazol, 2-[3'- tert.-Butyl-2'-hydroxy-5'-(2-isooctyloxycarbonylethyl)phenyl]- benztriazol, 2,2'-Methylen-bis[4-(1,1,3,3-tetramethylbutyl)-6- benztriazol-2-yl-phenol], das vollveresterte Produkt von 2-[3'- tert.-Butyl-5'-(2-methoxycarbonylethyl)-2'-hydroxyphenyl]-2H-benztriazol mit Polyethylenglycol 300, [R-CH2CH2-COO(CH2)3-]2 mit R gleich 3'-tert.-Butyl-4-hydroxy-5'-2H-benztriazol-2-ylphenyl, 2-[2'-Hydroxy-3'-(α,α-dimethylbenzyl)-5'-(1,1,3,3-tetramethylbutyl)phenyl]benztriazol, 2-[2'-Hydroxy-3'-(1,1,3,3-tetramethylbutyl)-5'-(a,a-dimethylbenzyl)phenyl]benztriazol;
Benzylidencampher oder seine Derivate, wie sie z. B. in der DE-A 38 36 630 genannt sind, z.B. 3-Benzylidencampher, 3(4'-Methylbenzyliden)d-1-campher;
α-(2-Oxoborn-3-yliden)toluol-4-sulfonsäure oder ihre Salze, N,N,N-Trimethyl-4-(2-oxoborn-3-ylidenmethyl)anilinium-monosulfat;
Dibenzoylmethane, wie z.B. 4-tert.-Butyl-4'-methoxydibenzoylmethan;
2,4,6-Triaryltriazin-Verbindungen wie 2,4,6-Tris-{N-[4-(2-ethylhex-1-yl)oxycarbonylphenyl]amino}-1,3,5-triazin, 4,4'-((6- (((tert.-Butyl)aminocarbonyl)phenylamino)-1,3,5-triazin-2,4- diyl)imino)bis(benzoesäure-2'-ethylhexylester);
2-(2-Hydroxyphenyl)-1,3,5-triazine, wie z.B. 2,4,6-Tris(2-hydroxy-4-octyloxyphenyl)1,3,5-triazin, 2-(2-Hydroxy-4-octyloxyphenyl)-4,6-bis(2,4-dimethylphenyl)-1,3,5- triazin, 2-(2,4-Dihydroxyphenyl)-4,6-bis(2,4-dimethylphenyl)-1,3,5-triazin, 2,4-Bis- (2-hydroxy-4-propyloxyphenyl)-6-(2,4-dimethylphenyl)-1,3,5-triazin, 2-(2-Hydroxy-4- octyloxyphenyl)-4,6-bis(4-methylphenyl)- 1,3,5-triazin, 2-(2-Hydroxy-4-dodecyloxyphenyl)-4,6-bis(2,4-dimethylphenyl)-1,3,5-triazin, 2-[2-Hydroxy-4-(2-hydroxy-3-butyloxypropyloxy)phenyl]-4,6-bis(2,4-dimethylphenyl)-1,3,5-triazin, 2-[2-Hydroxy-4-(2-hydroxy-3-octyloxypropyloxy)phenyl]-4,6-bis- (2,4-di-methylphenyl)-1,3,5-triazin, 2-(2-Hydroxy-4-tridecyloxyphenyl)-4,6-bis(2,4-dimethylphenyl)-1,3,5-triazin, 2-[4-(Dodecyloxy/tridecyloxy-2-hydroxypropoxy)-2-hydroxyphenyl]-4,6-bis(2,4- dimethylphenyl)-1,3,5-triazin, 2-[2-Hydroxy-4(2-hydroxy-3-dodecyloxypropoxy)phenyl]-4,6-bis-(2,4-dimethylphenyl)-1,3,5-triazin, 2-(2-Hydroxy-4-hexyloxyphenyl)-4,6-diphenyl-1,3,5-triazin, 2-(2- Hydroxy-4-methoxyphenyl)4,6-diphenyl-1,3,5-triazin, 2,4,6-Tris[2- hydroxy-4-(3-butoxy-2-hydroxypropoxy)phenyl]-1,3,5-triazin, 2-(2-Hydroxyphenyl)-4-(4-methoxyphenyl)-6-phenyl-1,3,5-triazin, 2-{2-Hydroxy-4-[3-(2-ethylhexyl-1-oxy)-2-hydroxypropyloxy]phenyl}-4,6- bis(2,4-dimethylphenyl)-1,3,5-triazin.
Geeignete optionale weitere UV-Absorber kann man der Schrift Cosmetic Legislation, Vol.1, Cosmetic Products, European Commission 1999, S. 64-66 entnehmen, auf die hiermit Bezug genommen wird.
Geeignete optionale weitere UV-Absorber werden außerdem in den Zeilen 14 bis 30 ([0030]) auf Seite 6 der EP 1 191 041 A2 beschrieben. Auf diese wird vollinhaltlich Bezug genommen.
Die optionalen weiteren UV-Absorber werden, wenn vorhanden, in der Regel in einer Menge von 0,01 Gew.-% bis 10 Gew.-% bezogen auf die Gesamtmenge an unbelebtem organischen Material oder Kunststoff eingesetzt. Bevorzugt werden von 0,05 bis 8 Gew.-% UV-Absorber eingesetzt, ganz bevorzugt von 0,1 bis 7 Gew.-%, insbesondere von 0,1 bis 5 Gew.%.

Diese optionalen Additive werden bei der Herstellung der unbelebten organischen Materialien, insbesondere der Kunststoffe oder Kunstoff-Formkörper oder im Rahmen optionaler zusätzlicher Schritte des Verfahrens zugesetzt.

In einer weiteren bevorzugten Ausführungsform der erfindungsgemäßen Verwendung werden die oben aufgezählten Benztriazolderivate als UV-Absorber in Textilien, bevorzugt in Badebekleidung, Oberbekleidung oder Outdoor-Bekleidung, eingesetzt.

In einer weiteren bevorzugten Ausführungsform der erfindungsgemäßen Verwendung werden die oben aufgezählten Benztriazolderivate als UV-Absorber in kosmetischen Zubereitungen eingesetzt.

In einer weiteren bevorzugten Ausführungsform der erfindungsgemäßen Verwendung werden die oben aufgezählten Benztriazolderivate als Lichtschutzmittel und Stabilisatoren für Kunststoffe eingesetzt.

Ein weiterer Gegenstand der Erfindung ist ein Verfahren zur Stabilisierung von Kunststoffen gegen die Einwirkung von Licht, optional in Zusammenhang mit der zusätzlichen Einwirkung von Sauerstoff auf den Kunststoff, dadurch gekennzeichnet, dass man hierzu die oben aufgezählten Benztriazolderivate verwendet. Hierbei werden den Kunststoffen von 0,01 bis 10 Gew.-% bezogen auf die Menge des Kunststoffs, von den oben aufgezählten Benztriazolderivaten zugesetzt. In einer Ausführungsform werden bevorzugt von 0,05 bis 1 Gew.-% Benztriazolderivate dem Kunststoff zugesetzt. In einer anderen Ausführungsform werden bevorzugt von 5 bis 8 Gew.-% Benztriazolderivate dem Kunststoff zugesetzt.

Ein weiterer Gegenstand der Erfindung sind gegen die Einwirkung von Licht, Sauerstoff und Wärme stabilisierte unbelebte organische Materialien, insbesondere Kunststoffe, enthaltend von 0,01 bis 10 Gew.-% bezogen auf die Menge des unbelebten organischen Materials, von den oben aufgezählten Benztriazolderivaten. In einer Ausführungsform sind bevorzugt von 0,05 bis 1 Gew.-% Benztriazolderivate im Kunststoff enthalten. In einer anderen Ausführungsform sind bevorzugt von 5 bis 8 Gew.-% Benztriazolderivate im Kunststoff enthalten.

Ein weiterer Gegenstand der Erfindung sind Benztriazolderivate der allgemeinen Formel (la), (Ib) und (Ic) bei denen die Gruppe A einem n-valenten organischen Radikal der allgemeinen Formeln (III) entspricht. Die Bedeutung der restlichen Symbole und Indizes entspricht der Bedeutung in den Formeln (la), (Ib), (Ic) und (III). Ganz bevorzugt ist hierbei n eine ganze Zahl aus dem Bereich von 3 bis 10, insbesondere von 3 bis 6. Ganz bevorzugt ist hierbei q eine ganze Zahl aus dem Bereich von 2 bis 10, besonders bevorzugt aus dem Bereich von 2 bis 4, insbesondere bevorzugt 2 oder 3, insbesondere 2. Ganz bevorzugt ist R⁷⁶ hierbei H, Aryl, C₁-C₃₀-Alkyl, bevorzugt C₂-C₂₀-Alkyl, besonders bevorzugt C₁₀-C₂₀-Alkyl, insbesondere C₁₈-Alkyl oder C₂₀-Alkyl.

Ein weiterer Gegenstand der Erfindung sind Benztriazolderivate der allgemeinen Formel (la), (Ib) und (Ic) bei denen die Gruppe A einem n-valenten organischen Radikal der allgemeinen Formeln (IIIa) entspricht. Die Bedeutung der restlichen Symbole und Indizes entspricht der Bedeutung in den Formeln (Ia), (Ib), (Ic) und (IIIa). Ganz bevorzugt ist hierbei in Formel (IIIa) n eine ganze Zahl aus dem Bereich von 5 bis 10, insbesondere von 5 bis 6. Ganz bevorzugt ist m eine ganze Zahl von 0 bis 5, insbesondere 0. Ganz bevorzugt ist R⁸⁷ ein C₁-C₁₀-Alkan, insbesondere ein C₁-C₅-Alkan bei dem n Wasserstoffe durch eine Bindung ersetzt sind.

Insbesondere bevorzugt sind hierbei Benztriazolderivate der er allgemeinen Formel (la), (Ib) und (Ic) bei denen die Gruppe A einem n-valenten organischen Radikal der allgemeinen Formeln (IIIa) entspricht und n = 5, 6, ist, R⁸⁷ ein C₁-C₁₀-Alkan, insbesondere ein C₁-C₅-Alkan bei dem n Wasserstoffe durch eine Bindung ersetzt sind, ist und R², R¹², R²³O sind.

Ein weiterer Gegenstand der Erfindung sind Benztriazolderivate der allgemeinen Formel (la), (Ib) und (Ic) bei denen die Gruppe A einem n-valenten organischen Radikal der allgemeinen Formeln (IIIb) entspricht. Die Bedeutung der restlichen Symbole und Indizes entspricht der Bedeutung in den Formeln (la), (Ib), (Ic) und (IIIb). Ganz bevorzugt ist hierbei in Formel (IIIb) n eine ganze Zahl aus dem Bereich von 5 bis 10, insbesondere von 5 bis 6. Ganz bevorzugt ist m eine ganze Zahl von 0 bis 5, insbesondere 2 oder 3. Ganz bevorzugt ist R⁹⁷ ein C₁-C₁₀-Alkan, insbesondere ein C₁-C₅-Alkan bei dem n Wasserstoffe durch eine Bindung ersetzt sind.

Bevorzugt beträgt das Molekulargewicht (Zahlenmittel Mn) der Benztriazolderivate der allgemeinen Formel (I), (la), (Ib) oder (Ic) von 1100 g/mol bis 5000 g/mol, besonders bevorzugt von 1500 bis 5000 g/mol, insbesondere von 2000 bis 5000 g/mol.

Ein weiterer Gegenstand der Erfindung sind nach dem bevorzugten Verfahren hergestellte Benztrizolderivate.

Die vorliegende Erfindung stellt UV-Absorber bzw. Stabilisatoren zur Verfügung die aus unbelebten organischen Materialien, insbesondere Kunststoffe nur in sehr geringem Ausmaß austreten. Die Unterdrückung der Migration bietet dabei Vorteile wie beispielsweise eine verlängerte Stabilisierungswirkung oder eine Vermeidung von die Gesundheit gefährdenden Auswirkungen der Stoffe, beispielsweise bei Kontakt der Stoffe mit Lebensmitteln.

Die Erfindung wird durch die Beispiele näher erläutert ohne dass die Beispiele den Gegenstand der Erfindung einschränken.

### Beispiele:

Unterschiedliche Benzotriazolderivate wurden nach den folgenden Vorschriften synthetisiert.

Daten zur molekular Masse, Adsorptionsparameter und Schmelzpunkte sind in der Tabelle 1 zu finden.
Raumtemperatur: 21°C

### Beispiel 1: Oligoetheramine

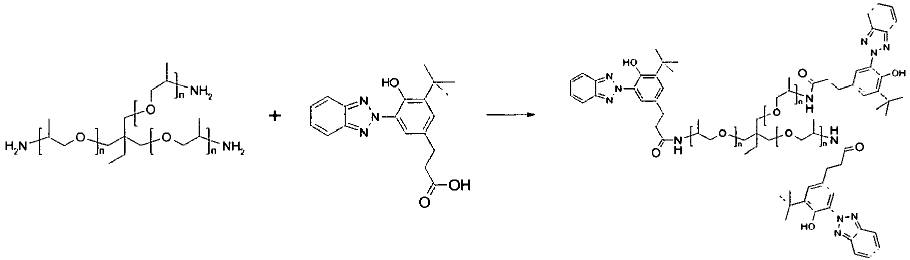

Unter Stickstoffatmosphäre wurden 13,6 g (0,04 mol) 3-(3-Benzotriazol-2-yl-5-tert-butyl-4-hydroxy-phenyl)-propionsäure und 6,3 g (0,0143 mol) Polyetheramin (M = 440g/mol) bei Raumtemperatur vorgelegt. Der Ansatz wurde 5h bei 200°C gerührt, wobei das entstehende Reaktionswasser ausgekreist wurde. Beim Abkühlen auf Raumtemperatur wurde das Reaktionsgemisch fest und man erhielt 17,5g des kristallinen Produkts (84% Ausbeute).

### Beispiel 2: Maleinsäureanhydrid (MSA)-Derivate

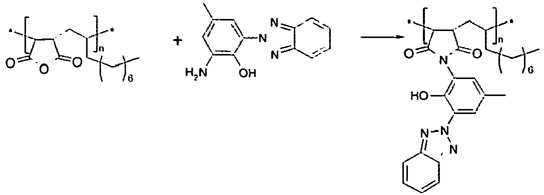

Unter Stickstoffatmosphäre wurden 8,65g (0,02 mol) MSA-α-Olefin (n = 1,8) ( 50,6% ig in Solvesso 150 gelöst) und 4,81g (0,02 mol) 2-Amino-6-benzotriazol-2-yl-4-methylphenol in 40 ml Solvesso 150 (Naphtha-Schnitt: Mischung von schweren aromatischen Kohlenwasserstoffen) bei Raumtemperatur vorgelegt. Der Ansatz wurde 20h bei 175°C unter Rückfluß gerührt, wobei das entstehende Reaktionswasser ausgekreist wurde. Beim Abkühlen auf Raumtemperatur fiel das Produkt aus. Der Rückstand wurde abgesaugt und mit Petrolether gewaschen. Das Lösungsmittel wurde von der organischen Phase im Vakuum entfernt. Der erhaltene Rückstand wurde in Essigester gelöst und mit Aktivkohle 1h bei RT ausgerührt. Man filtrierte die Aktivkohle ab und entfernte das Lösungsmittel im Vakuum. Man erhielt 4,37g kristallines Produkt.

### Beispiel 3: Weitere MSA-Derivate

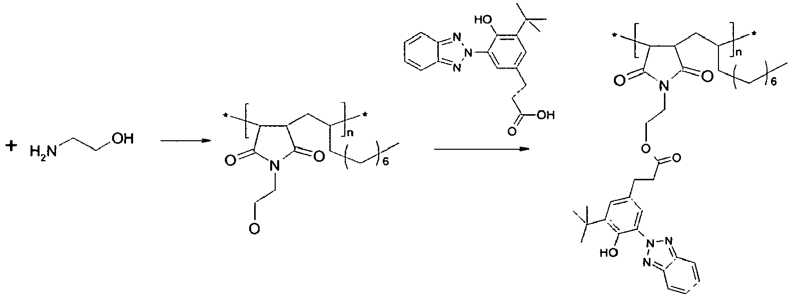

Unter Stickstoffatmosphäre wurden 8,65g (0,02 mol) MSA-α-Olefin CP Decen (n = 1,8) und 2,4 g (0,04 mol) Ethanolamin bei Raumtemperatur vorgelegt und anschließend 2h bei 163°C unter Rückfluß gerührt, wobei das entstehende Reaktionswasser ausgekreist wurde. Der Ansatz wurde auf 120°C abgekühlt. Es wurden 13,6 g ( 0,04 mol ) 3-(3-Benzotriazol-2-yl-5-tert-butyl-4-hydroxy-phenyl)-propionsäure und 0,2 g p-Toluolsulfonsäure Monohydrat zugegeben. Das Reaktionsgemisch wurde 6h bei 163°C gerührt und anschließend auf Raumtemperatur abgekühlt. Es wurden 200 ml Dichlormethan und 10g Kieselgel zugegeben. Der Ansatz wurde 1 h bei Raumtemperatur gerührt. Das Kieselgel wurde abgetrennt und das Lösungsmittel im Vakuum entfernt. Es wurden 11,7g kristallines Produkt erhalten.

### Beispiel 4: Carbonsäurederivate

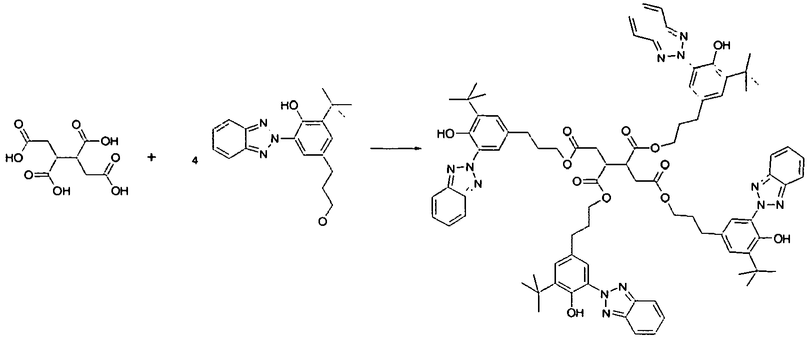

Unter Stickstoffatmosphäre wurden 2,3 g (0,01 mol) 1,2,3,4-Butan-tetracarbonsäure, 13,0 g (0,04 mol) 2-(2'-Hydroxy-5'hydroxypropylphenyl)-2H-benztriazol und 0,15 ml (0,002 mol) Methansulfonsäure in 75 ml Solvesso 100 (Naphtha-Schnitt: Mischung von leichten aromatischen Kohlenwasserstoffen) bei Raumtemperatur vorgelegt und anschließend 24h bei 165°C unter Rückfluß gerührt, wobei das entstehende Reaktionswasser ausgekreist wurde. Der Ansatz wurde auf Raumtemperatur abgekühlt und das Lösungsmittel im Vakuum entfernt. Zum Rückstand wurden 500 ml Petrolether zugegeben. Der Ansatz wurde 1 h bei Raumtemperatur gerührt. Die Suspension wurde über eine Fritte abgesaugt. Der Rückstand wurde mit wenig Petrolether gewaschen und bei 60°C im Vakuumschrank getrocknet. Man erhielt 13,8g farbloses kristallines Produkt (94% Ausbeute).

### Beispiel 5: Cyanursäure-Derivate

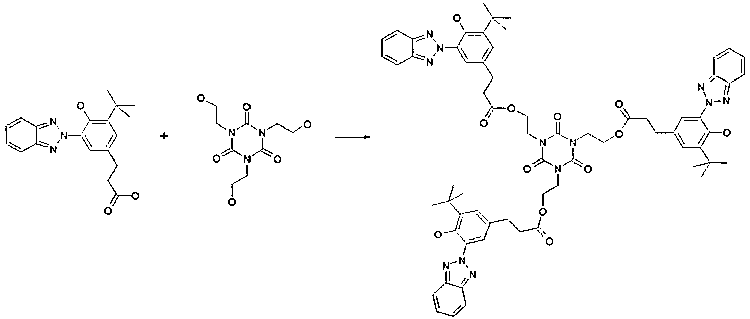

Unter Stickstoffatmosphäre wurden 6,5 g (0,0025 mol)1,3,5-Tris-(2-hydroxyethyl)cyanursäure, 25,5 g (0,075 mol) 3-(3-Benzotriazol-2-yl-5-tert-butyl-4-hydroxyphenyl)-propionsäure und 0,5 ml (0,009 mol) Methansulfonsäure in 150 ml Xylol bei Raumtemperatur vorgelegt und anschließend 4 Tage bei 140°C ( Rückfluß ) gerührt, wobei das entstehende Reaktionswasser ausgekreist wurde. Der Ansatz wurde auf Raumtemperatur abgekühlt und das Lösungsmittel im Vakuum entfernt. Der erhaltene Rückstand wurde säulenchromatographisch (100 Toluol:2,5 Essigester) gereinigt. Man erhielt 16,2g kristallines Produkt (53% Ausbeute).

### Beispiel 6: Cyanurchlorid-Derivate

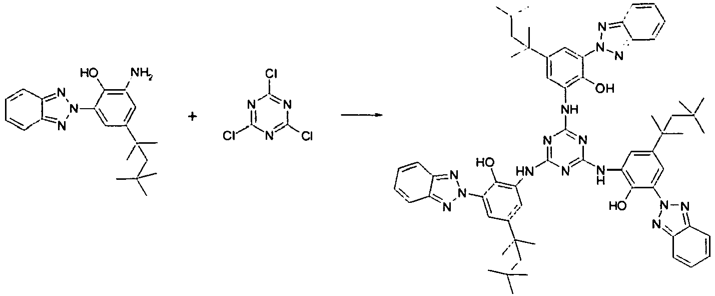

Unter Stickstoffatmosphäre wurden 1,8 g (0,01 mol) Cyanurchlorid, 10,15 g (0,03 mol) 2-Amino-6-benzotriazol-2-yl-4-tert.octyl-phenol und 2,5 g (0,03 mol) Natriumhydrogencarbonat in einem Lösungsmittelgemisch aus 15 ml Dimethylformamid (DMF) und 4,5 ml 1,4-Dioxan bei Raumtemperatur vorgelegt. Der Ansatz wurde 24h bei Rückfluß (102°C) gerührt und anschließend auf Raumtemperatur abgekühlt. Die Suspension wurde über eine Fritte abgesaugt. Der Rückstand wurde mit Wasser gewaschen und bei 50°C im Vakuumschrank getrocknet. Es wurden 9,32g eines kristallinen Produkts (85,5% Ausbeute) erhalten.

### Beispiel 7: Weitere Cyanurchlorid-Derivate

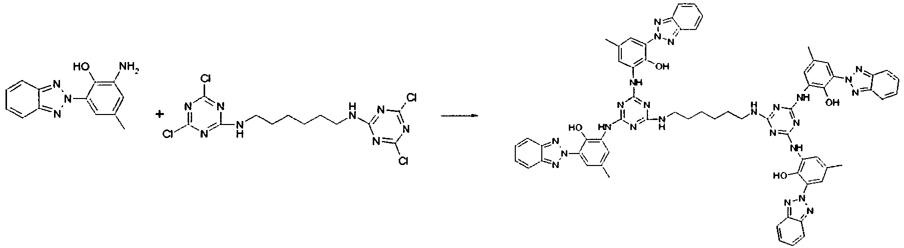

Unter Stickstoffatmosphäre wurden 1,29 g (0,003 mol) N,N'-bis-(4,6-dichloro-[1,3,5]triazin-2-yl)-hexane-1,6-diamine, 3,36 g (0,014 mol) 2-amino-6-benzotriazol-2-yl-4-methyl-phenol und 0,83 g (0,01 mol) Natriumhydrogencarbonat in 20 ml N-Methyl-2-pyrrolidon bei Raumtemperatur vorgelegt. Der Ansatz wurde 24h bei 160°C gerührt und anschließend auf RT abgekühlt. Zum Reaktionsgemisch wurden 20 ml Methanol zugegeben. Es wurde 1h bei Raumtemperatur nachgerührt. Die Suspension wurde über eine Fritte abgesaugt. Der Rückstand wurde zunächst mit Methanol und abschließend mit Wasser gewaschen. Der Rückstand wurde bei 50°C im Vakuumschrank getrocknet. Es wurden 9,97g eines kristallinen Produkts (90% Ausbeute) erhalten.

### Beispiel 8: Derivate mit propoxyliertem Triol

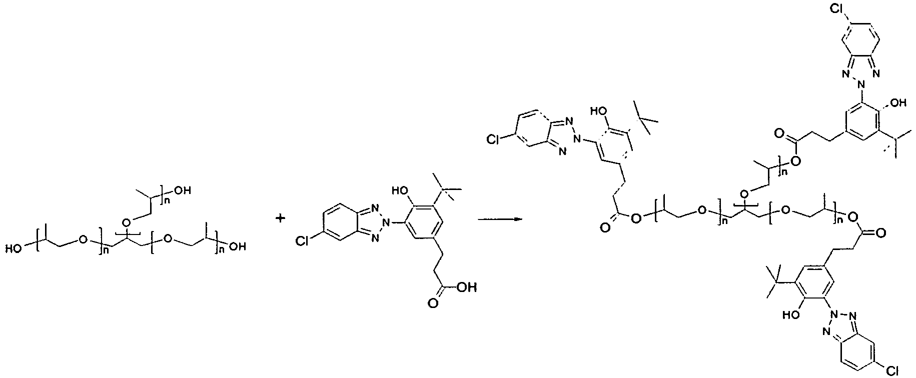

Unter Stickstoffatmosphäre wurden 14,9 g (0,04 mol) 2-(5-Chlor-2H-benzotriazol-2-yl)-6-tert-butyl-4-hydroxy-phenyl-propionsäure, 5,7 g (0,04 mol) polyetherpolyol (Mw = 420) und 0,35 ml (0,005 mol) Methansulfonsäure in 200 ml Xylol bei Raumtemperatur vorgelegt und anschließend 24h bei 140°C (Rückfluß) gerührt, wobei das entstehende Reaktionswasser ausgekreist wurde. Der Ansatz wurde auf Raumtemperatur abgekühlt und das Lösungsmittel im Vakuum entfernt. Der erhaltene Rückstand wurde säulenchromatographisch (100 Toluol:2,5 Essigester) gereinigt. Man erhielt 16,8g des Produkts (90% Ausbeute).

### Beispiel 9: Diolderivate

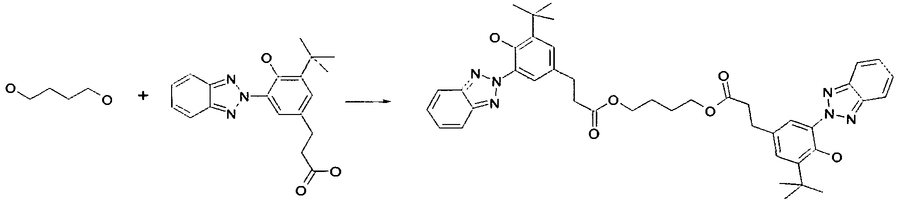

Unter Stickstoffatmosphäre wurden 23,8 g (0,07 mol) 3-(3-Benzotriazol-2-yl-5-tert-butyl-4-hydroxy-phenyl)-propionsäure, 3,15 g (0,035 mol) 1,4-Butandiol und 0,5 ml (0,008 mol) Methansulfonsäure in 100 ml Xylol bei Raumtemperatur vorgelegt und anschließend 2 Tage bei 140°C (Rückfluß) gerührt, wobei das entstehende Reaktionswasser ausgekreist wurde. Der Ansatz wird auf Raumtemperatur abgekühlt und das Lösungsmittel im Vakuum entfernt. Der erhaltene Rückstand wird säulenchromatographisch (100 Toluol:2,5 Essigester) gereinigt. Es wurden 20,7g des kristallinen Produkts (80,5% Ausbeute) erhalten.

### Beispiel 10: Trishydroxymethylpropan-Derivate

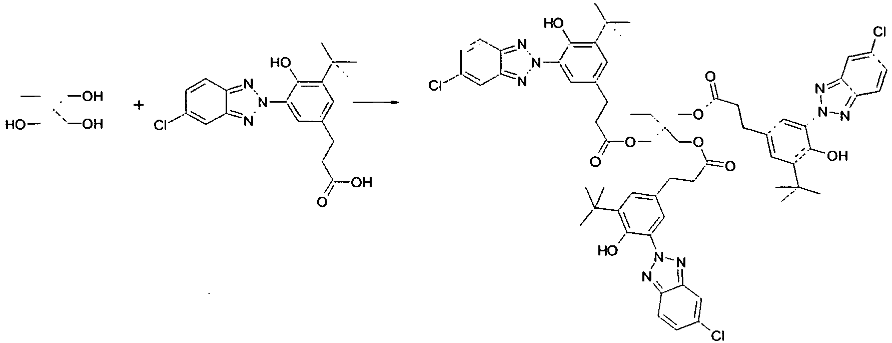

Unter Stickstoffatmosphäre wurden 16,8 g (0,045 mol) 2-(5-Chlor-2H-benzotriazol-2-yl)-6-tert-butyl-4-hydroxy-phenyl-propionsäure, 2,05 g (0,015 mol) 1,1,1-Tris-(hydroxymethyl)-propan und 0,58 ml (0,008 mol) Methansulfonsäure in 150 ml Xylol bei Raumtemperatur vorgelegt und anschließend 4 Tage bei 140°C (Rückfluß) gerührt, wobei das entstehende Reaktionswasser ausgekreist wurde. Der Ansatz wurde auf Raumtemperatur abgekühlt und das Lösungsmittel im Vakuum entfernt. Der erhaltene Rückstand wurde säulenchromatographisch (100 Toluol:2,5 Essigester) gereinigt. Man erhielt 11,2g des kristallinen Produkts (62% Ausbeute).

### Beispiel 11: Pentaerythritderivate

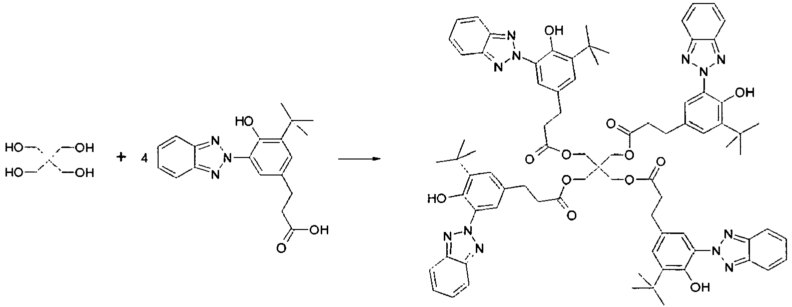

Unter Stickstoffatmosphäre wurden 17,0 g (0,05 mol) 3-(3-Benzotriazol-2-yl-5-tert-butyl-4-hydroxy-phenyl)-propionsäure, 1,7 g (0,0125 mol) Pentaerythrit und 0,50 ml (0,008 mol) Methansulfonsäure in 100 ml Xylol bei Raumtemperatur vorgelegt und anschließend 3 Tage bei 140°C (Rückfluß) gerührt, wobei das entstehende Reaktionswasser ausgekreist wurde. Der Ansatz wurde auf Raumtemperatur abgekühlt und das Lösungsmittel im Vakuum entfernt. Der erhaltene Rückstand wurde säulenchromatographisch (100 Toluol:2,5 Essigester) gereinigt. Man erhielt 10,7g des kristallinen Produkts (60% Ausbeute).

### Beispiel 12: Weitere Pentaerythritderivate

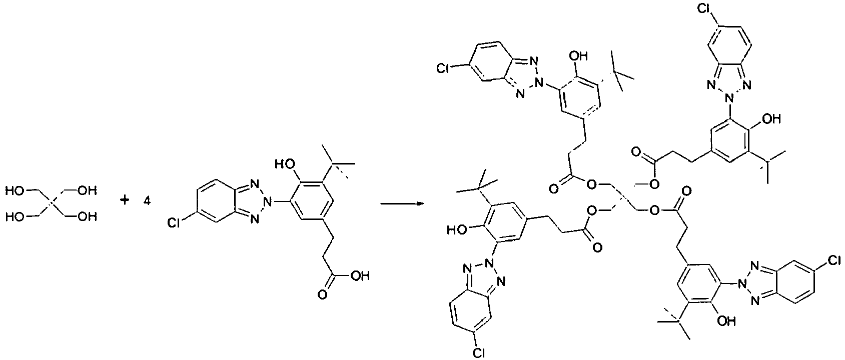

Unter Stickstoffatmosphäre wurden 18,7 g ( 0,05 mol ) 2-(5-Chlor-2H-benzotriazol-2-yl)-6-tert-butyl-4-hydroxy-phenyl-propionsäure, 1,7 g (0,0125 mol) Pentaerythrit und 0,50 ml (0,008 mol) Methansulfonsäure in 100 ml Xylol bei Raumtemperatur vorgelegt und anschließend 3 Tage bei 140°C (Rückfluß) gerührt, wobei das entstehende Reaktionswasser ausgekreist wurde. Der Ansatz wurde auf Raumtemperatur abgekühlt und das Lösungsmittel im Vakuum entfernt. Der erhaltene Rückstand wurde säulenchromatographisch (100 Toluol:2,5 Essigester ) gereinigt. Man erhielt 10,1g des kristallinen Produkts (51 % Ausbeute).

In der folgenden Tabelle 1 sind physikalisch-chemische Daten für die Verbindungen aus den oben genanten Beispielen und für die Vergleichssubstanzen zusammengefasst. Die UV-Spektren wurden in Methylenchlorid (außer Beispiel 7: in NMP) in einer Konzentration von 0,5g/Liter gemessen. Die Onset-Temperatur (Abbau-Temperatur: T-Onset) wurde mit Hilfe von Thermogravimetrie unter Luft gemessen, zwischen 30°C und 600°C (Heizrate = 10°C pro Minute). Der T-Onset-Wert ist ein Maß für die Temperatur-Stabilität der Substanz und sollte mindestens die Temperatur erreichen bei der eine Verarbeitung des Polymers erfolgt.

Die Substanzen aus den Beispielen zeigten insgesamt eine Absorption im UV vergleichbar mit den Vergleichssubstanzen. Bei einem wesentlich höheren Molekulargewicht zeigen diese Substanzen, einen vergleichbaren oder höheren Extinktionskoeffizienten und eine vergleichbaren oder höheren T-Onset-Wert relativ zu den Vergleichssubstanzen.

**Tabelle 1**

| Beispiel Nummer | Peakposition (nm) | Extinktionskoeffizient [l/cm*g] | T-Onset (°C) | Molmasse (g/mol) |
|---|---|---|---|---|
| 1 | 342 | 30,6455 | 386.3 | ca.1420 |
| | 302 | 31,1545 | | |
| 2 | 336 | 24,2488 | 386.7 | >1100 |
| | 298 | 23,9558 | | |
| 3 | 342 | 21,4761 | 379.9 | >1100 |
| | 302 | 21,4652 | | |
| 4 | 342 | 40,1960 | 356.2 | 1463,76 |
| | 302 | 40,9720 | | |
| 5 | 342 | 35,5310 | 379.2 | 1225,38 |
| | 302 | 35,2793 | | |
| 6 | 324 | 66,5000 | 378.2 | 1090,4 |
| | 276 | 70,6900 | | |
| 7 | 314 | 54.8833 | 419.2 | 1227,33 |
| 8 | 350 | 31,2560 | 328.5 | ca.1400 |
| | 310 | 27,4400 | | |
| 9 | 342 | 41,4160 | 355.5 | 732,89 |
| | 302 | 40,9080 | | |
| 10 | 350 | 41,1840 | 376.5 | 1201,66 |
| | 310 | 35,7960 | | |
| 11 | 342 | 42,2250 | 392.3 | 1421,68 |
| | 302 | 41,9333 | | |
| 12 | 348 | 41,5960 | 377.1 | 1559,46 |
| | 310 | 36,1320 | | |
| Vergleich 1 | 342 | 31,9406 | 313.9 | 451,61 |
| | 302 | 31,7094 | | |
| Vergleich 2 | 340 | 72,3600 | 228.7 | 225,25 |
| | 298 | 61,1000 | | |
| Vergleich 3 | 340 | 50,0762 | 255.7 | 323,44 |
| | 300 | 45,1762 | | |

### Vergleich 1: Tinuvin 384-2 ® (Ciba)

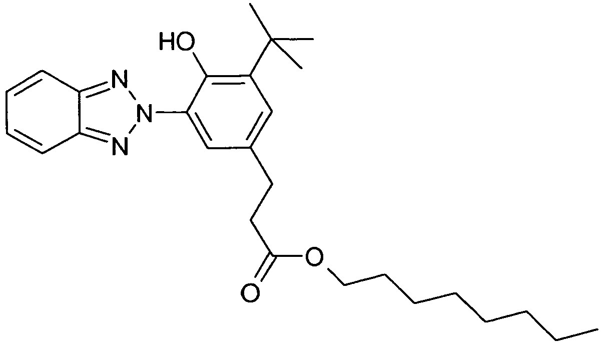

### Vergleich 2: Tinuvin P ® (Ciba)

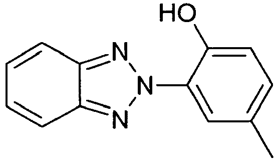

### Vergleich 3: Tinuvin 329 ® (Ciba)

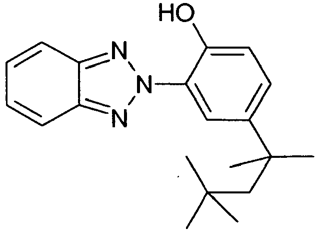

## Patentansprüche

1. Verwendung von Benztriazolderivaten der allgemeinen Formel (I) 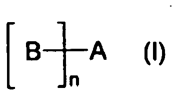 wobei
B eine gegebenenfalls substituierte 2-(2-Hydroxyphenyl)-2H-benztriazolgruppe,
n eine ganze Zahl aus dem Bereich von 3 bis 20 und
A ein n-valentes organisches Radikal
ist,
als UV-Absorber oder Stabilisator in unbelebten organischen Materialien,
wobei A einem n-valenten organischen Radikal der allgemeinen Formeln (III), (IIIa) oder (IIIb) oder einem tri-valenten organischen Radikal der allgemeinen Formel (IIId) entspricht 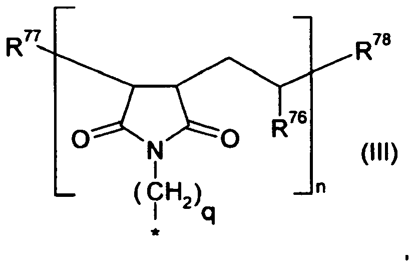 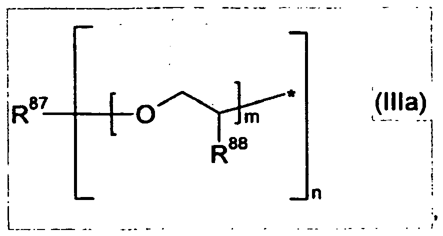 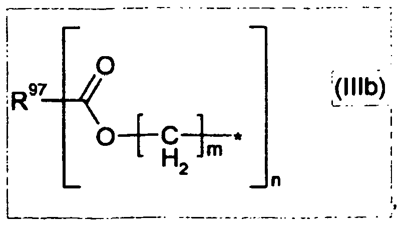 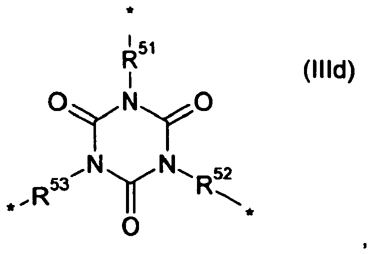 mit
m unabhängig voneinander eine ganze Zahl aus dem Bereich 0 bis 10,
q ganze Zahl aus dem Bereich von 0 bis 10,
R⁵¹, R⁵², R³³ unabhängig voneinander, C₁-C₂₀-Alkylen,
R⁷⁶ H, Aryl, C₁-C₃₀-Alkyl,
R⁷⁷ H, C₁-C₂₀-Alkyl, C₁-C₂₀-Alkoxy,
R⁷⁸ H, C₁-C₂₀-Alkyl, C₁-C₂₀-Alkoxy,
R⁸⁷ lineares oder verzweigtes C₁-C₂₀-Alkan, bei dem n Wasserstoffe durch eine Bindung ersetzt sind,
R⁸⁸ H, linear oder verzweigtes C₁-C₂₀-Alkyl,
R⁹⁷ lineares oder verzweigtes C₁-C₂₀-Alkan, bei dem n Wasserstoffe durch eine Bindung ersetzt sind,
und
wobei * die Anbindung des Benztriazolderivats an das n-valente organische Radikal der Formeln (III), (IIIa) oder (IIIb) oder an das tri-valente organische Radikal (IIId) bezeichnet.

2. Verwendung nach Anspruch 1, wobei Benztriazolderivate der allgemeinen Formel (Ia) 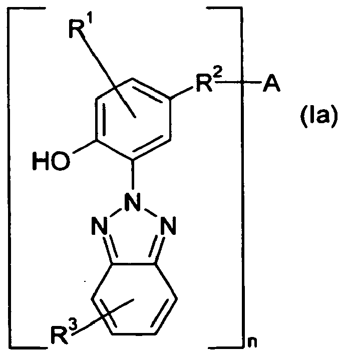 mit
n eine ganze Zahl aus dem Bereich von 3 bis 20,
R¹ H, C₁-C₂₀-Alkyl, Arylalkyl, C₅-C₂₀-Alkoxycarbonylalkyl, C₃-C₁₅-Cycloalkyl,
R3 H, Halogen, CF₃, COOR⁴,
R⁴ H, C₁-C₂₀-Alkyl,
R² eine Einfachbindung, O, NR⁷ oder eine Gruppe der allgemeinen Formel (IIa) 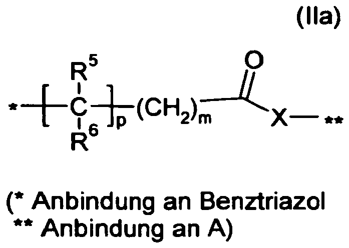
m eine ganze Zahl aus dem Bereich von 0 bis 4,
p 0 oder 1,
A ein n-valentes organisches Radikal darstellt,
R⁵ H, C₁-C₂₀-Alkyl,
R⁶ H, C₁-C₂₀-Alkyl,
X O, NR⁷,
R⁷ H, C₁-C₂₀-Alkyl,
eingesetzt werden.

3. Verwendung nach Anspruch 1, wobei Benztriazolderivate der allgemeinen Formel (Ib) 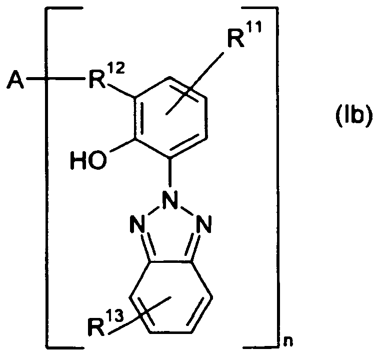 mit
R¹¹ H, C₁-C₂₀-Alkyl, Arylalkyl, C₅-C₂₀-Alkoxycarbonylalkyl, C₃-C₁₅-Cycloalkyl,
R¹³ H, Halogen, CF₃, COOR¹⁴,
R¹⁴ H, C₁-C₂₀-Alkyl.
R¹² eine Einfachbindung, O, NR⁷ oder eine Gruppe der allgemeinen Formel (IIa) gemäß Anspruch 2
eingesetzt werden.

4. Verwendung nach Anspruch 1, wobei Benztriazolderivate der allgemeinen Formel (Ic) 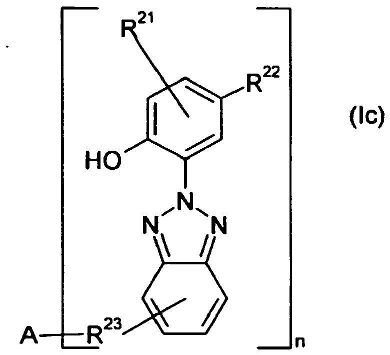 mit
R²¹ H, C₁-C₂₀-Alkyl, Arylalkyl, C₅-C₂₀-Alkoxycarbonylalkyl, C₃-C₁₅-Cycloalkyl,
R²² H, C₁-C₂₀-Alkyl, COOR²⁴,
R²⁴ H, C₁-C₂₀-Alkyl,
R²³ eine Einfachbindung, O, NR⁷ oder eine Gruppe der allgemeinen Formel (IIa) gemäß Anspruch 2
eingesetzt werden.

5. Verwendung nach den Ansprüchen 1 bis 3, wobei das Molekulargewicht der Benztriazolderivate der allgemeinen Formel (I), (Ia) oder (Ib) von 1100 g/mol bis 5000 g/mol beträgt.

6. Verwendung von Benztriazolderivaten gemäß den Ansprüchen 1 bis 5 als UV-Absorber in Kunststoffen oder Wachsen.

7. Verwendung von Benztriazolderivaten gemäß den Ansprüchen 1 bis 5 als UV-Absorber in Kunststoff-Formkörpem.

8. Verwendung von Benztriazolderivaten gemäß den Ansprüchen 1 bis 5 als UV-Absorber in Textilien.

9. Verwendung von Benztriazolderivaten gemäß den Ansprüchen 1 bis 5 als UV-Absorber in kosmetischen Zubereitungen.

10. Verwendung von Benztriazolderivaten gemäß den Ansprüchen 1 bis 5 als Lichtschutzmittel und Stabilisatoren für Kunststoffe.

11. Verfahren zur Stabilisierung von Kunststoffen gegen die Einwirkung von Licht, optional in Zusammenhang mit der Einwirkung von Sauerstoff auf den Kunststoff, **dadurch gekennzeichnet, dass** man hierzu Benztriazolderivate gemäß den Ansprüchen 1 bis 5 verwendet.

12. Gegen die Einwirkung von Licht, Sauerstoff und Wärme stabilisierte unbelebte organische Materialien, enthaltend 0,01 bis 10 Gew.-% bezogen auf die Menge des unbelebten organischen Materials, von Benztriazolderivaten gemäß den Ansprüchen 1 bis 5.

13. Benztriazolderivate gemäß Anspruch 1, wobei A einem n-valenten organischen Radikal der Formel (III) entspricht und n eine ganze Zahl aus dem Bereich von 3 bis 20 ist.

14. Benztriazolderivate gemäß Anspruch 1, wobei A einem n-valenten organischen Radikal der Formel (IIIa) entspricht und n eine ganze Zahl aus dem Bereich von 5 bis 20 ist.

15. Benztriazolderivate gemäß Anspruch 1, wobei A einem n-valenten organischen Radikal der Formel (IIIb) entspricht und n eine ganze Zahl aus dem Bereich von 3 bis 20 ist.

## Claims

1. The use of benzotriazole derivatives of the general formula (I) 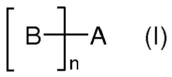 where
B is an optionally substituted 2-(2-hydroxyphenyl)-2H-benzotriazole group,
n is an integer from the range from 3 to 20 and
A is an n-valent organic radical,
as UV absorber or stabilizer in inanimate organic materials,
where A corresponds to an n-valent organic radical of the general formula (III), (IIIa) or (IIIb) or a trivalent organic radical of the general formula (IIId) 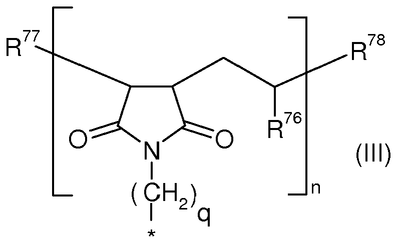 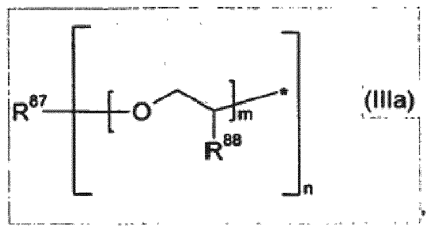 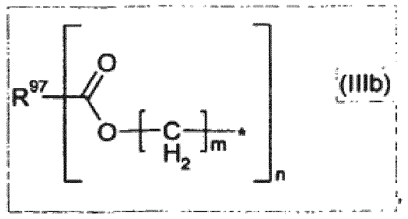 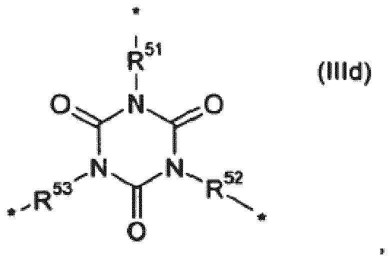 where
m independently of the others is an integer from the range 0 to 10,
q is an integer from the range from 0 to 10,
R⁵¹, R⁵², R⁵³ independently of one another are C₁-C₂₀-alkylene,
R⁷⁶ is H, aryl, C₁-C₃₀-alkyl,
R⁷⁷ is H, C₁-C₂₀-alkyl, C₁-C₂₀-alkoxy,
R⁷⁸ is H, C₁-C₂₀-alkyl, C₁-C₂₀-alkoxy,
R⁸⁷ is a linear or branched C₁-C₂₀-alkane in which n hydrogens are replaced by a bond,
R⁸⁸ is H, linear or branched C₁-C₂₀-alkyl,
R⁹⁷ is a linear or branched C₁-C₂₀-alkane in which n hydrogens are replaced by a bond,
and
where * designates the bonding of the benzotriazole derivative to the n-valent organic radical of the formula (III), (IIIa) or (IIIb) or to the trivalent organic radical (IIId).

2. The use according to claim 1, where benzotriazole derivatives of the general formula (Ia) 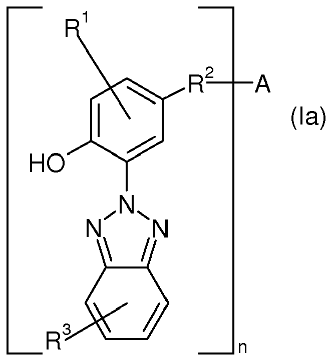 where
n is an integer from the range from 3 to 20,
R¹ is H, C₁-C₂₀-alkyl, arylalkyl, C₅-C₂₀-alkoxycarbonylalkyl, C₃-C₁₅-cycloalkyl,
R³ is H, halogen, CF₃, COOR⁴,
R⁴ is H, C₁-C₂₀-alkyl,
R² is a single bond, O, NR⁷ or a group of the general formula (IIa) 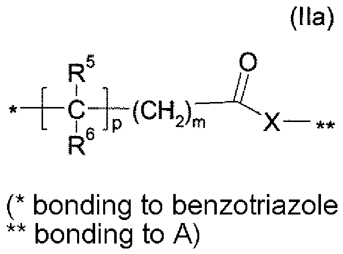
m is an integer from the range from 0 to 4,
p is 0 or 1,
A is an n-valent organic radical,
R⁵ is H, C₁-C₂₀-alkyl,
R⁶ is H, C₁-C₂₀-alkyl,
X is O, NR⁷,
R⁷ is H, C₁-C₂₀-alkyl,
are used.

3. The use according to claim 1, where benzotriazole derivatives of the general formula (Ib) 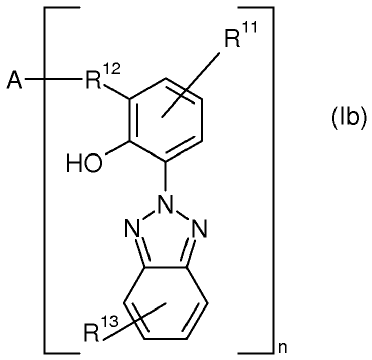 where
R¹¹ is H, C₁-C₂₀-alkyl, arylalkyl, C₅-C₂₀-alkoxycarbonylalkyl, C₃-C₁₅-cycloalkyl,
R¹³ is H, halogen, CF₃, COOR¹⁴,
R¹⁴ is H, C₁-C₂₀-alkyl,
R¹² is a single bond, O, NR⁷ or a group of the general formula (IIa) according to claim 2,
are used.

4. The use according to claim 1, where benzotriazole derivatives of the general formula (Ic) 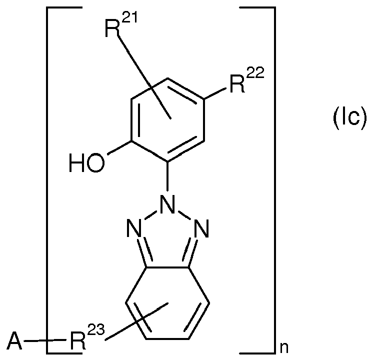 where
R²¹ is H, C₁-C₂₀-alkyl, arylalkyl, C₅-C₂₀-alkoxycarbonylalkyl, C₃-C₁₅-cycloalkyl,
R²² is H, C₁-C₂₀-alkyl, COOR²⁴,
R²⁴ is H, C₁-C₂₀-alkyl,
R²³ is a single bond, O, NR⁷ or a group of the general formula (IIa) according to claim 2
are used.

5. The use according to claims 1 to 3, where the molecular weight of the benzotriazole derivatives of the general formula (I), (Ia) or (Ib) is from 1100 g/mol to 5000 g/mol.

6. The use of benzotriazole derivatives according to claims 1 to 5 as UV absorbers in plastics or waxes.

7. The use of benzotriazole derivatives according to claims 1 to 5 as UV absorbers in plastic moldings.

8. The use of benzotriazole derivatives according to claims 1 to 5 as UV absorbers in textiles.

9. The use of benzotriazole derivatives according to claims 1 to 5 as UV absorbers in cosmetic preparations.

10. The use of benzotriazole derivatives according to claims 1 to 5 as photoprotective agents and stabilizers for plastics.

11. A method of stabilizing plastics against the effect of light, optionally in conjunction with the effect of oxygen on the plastic, which comprises using for this benzotriazole derivatives according to claims 1 to 5.

12. An inanimate organic material stabilized against the effect of light, oxygen and heat, comprising 0.01 to 10% by weight, based on the amount of the inanimate organic material, of benzotriazole derivatives according to claims 1 to 5.

13. A benzotriazole derivative according to claim 1, where A corresponds to an n-valent organic radical of the formula (III) and n is an integer from the range from 3 to 20.

14. A benzotriazole derivative according to claim 1, where A corresponds to an n-valent organic radical of the formula (Illa) and n is an integer from the range from 5 to 20.

15. A benzotriazole derivative according to claim 1, where A corresponds to an n-valent organic radical of the formula (IIIb) and n is an integer from the range from 3 to 20.

## Revendications

1. Utilisation de dérivés de benzotriazole de formule générale (I) 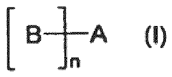 dans laquelle
B représente un groupe 2-(2-hydroxyphényl)-2H-benzotriazole éventuellement substitué,
n représente un nombre entier dans la plage allant de 3 à 20, et
A représente un radical organique n-valent,
en tant qu'absorbeur UV ou stabilisateur dans des matériaux organiques non vivants, A correspondant à un radical organique n-valent de formule générale (III), (IIIa) ou (IIIb) ou à un radical organique trivalent de formule générale (IIId) 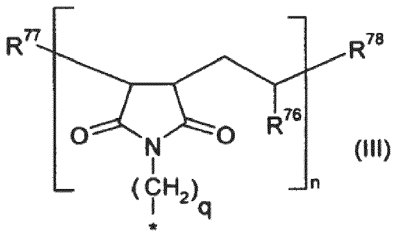 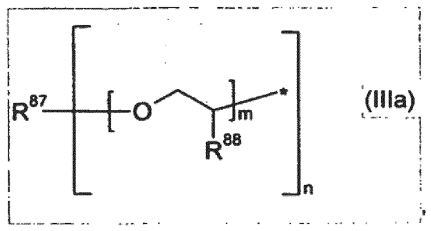 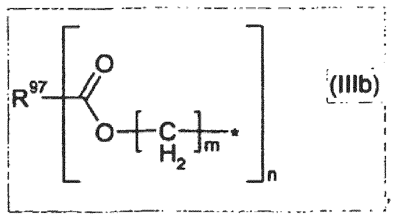 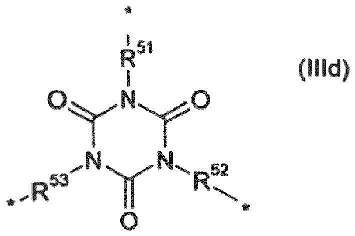 dans lesquelles
les m représentent indépendamment les uns des autres un nombre entier dans la plage allant de 0 à 10,
q représente un nombre entier dans la plage allant de 0 à 10,
R⁵¹, R⁵², R⁵³ représentent indépendamment les uns des autres alkylène en C₁-C₂₀,
R⁷⁶ représente H, aryle, alkyle en C₁-C₃₀,
R⁷⁷ représente H, alkyle en C₁-C₂₀, alcoxy en C₁-C₂₀,
R⁷⁸ représente H, alkyle en C₁-C₂₀, alcoxy en C₁-C₂₀,
R⁸⁷ représente un alcane en C₁-C₂₀ linéaire ou ramifié, dans lequel n hydrogènes sont remplacés par une liaison,
R⁸⁸ représente H, alkyle en C₁-C₂₀ linéaire ou ramifié,
R⁹⁷ représente un alcane en C₁-C₂₀ linéaire ou ramifié, dans lequel n hydrogènes sont remplacés par une liaison,
et
* représente la liaison du dérivé de benzotriazole au radical organique n-valent de formule (III), (IIIa) ou (IIIb) ou au radical organique trivalent (IIId).

2. Utilisation selon la revendication 1, dans laquelle des dérivés de benzotriazole de formule générale (Ia) 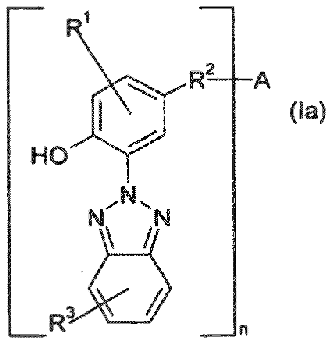 dans laquelle
n représente un nombre entier dans la plage allant de 3 à 20,
R¹ représente H, alkyle en C₁-C₂₀, arylalkyle, alcoxycarbonylalkyle en C₅-C₂₀, cycloalkyle en C₃-C₁₅,
R³ représente H, halogène, CF₃, COOR⁴,
R⁴ représente H, alkyle en C₁-C₂₀,
R² représente une simple liaison, O, NR⁷, ou un groupe de formule générale (IIa) 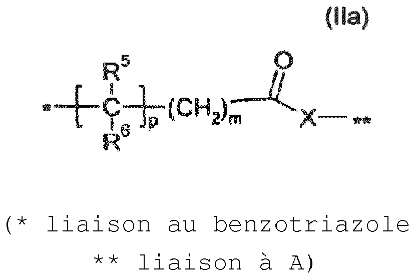
m représente un nombre entier dans la plage allant de 0 à 4,
p représente 0 ou 1,
A représente un radical organique n-valent,
R⁵ représente H, alkyle en C₁-C₂₀,
R⁶ représente H, alkyle en C₁-C₂₀,
X représente O, NR⁷,
R⁷ représente H, alkyle en C₁-C₂₀,
sont utilisés.

3. Utilisation selon la revendication 1, dans laquelle des dérivés de benzotriazole de formule générale (Ib) 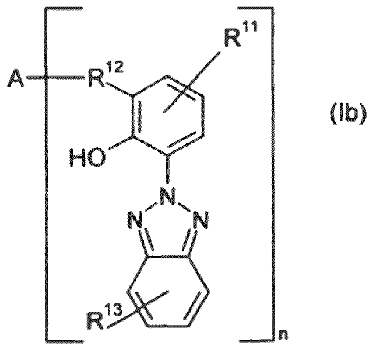 dans laquelle
R¹¹ représente H, alkyle en C₁-C₂₀, arylalkyle, alcoxycarbonylalkyle en C₅-C₂₀, cycloalkyle en C₃-C₁₅,
R¹³ représente H, halogène, CF₃, COOR¹⁴,
R¹⁴ représente H, alkyle en C₁-C₂₀,
R¹² représente une simple liaison, O, NR⁷, ou un groupe de formule générale (IIa) selon la revendication 2,
sont utilisés.

4. Utilisation selon la revendication 1, dans laquelle des dérivés de benzotriazole de formule générale (Ic) 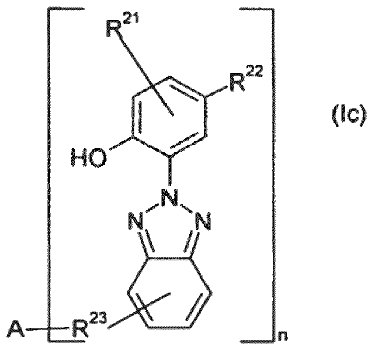 dans laquelle
R²¹ représente H, alkyle en C₁-C₂₀, arylalkyle, alcoxycarbonylalkyle en C₅-C₂₀, cycloalkyle en C₃-C₁₅,
R²² représente H, alkyle en C₁-C₂₀, COOR²⁴,
R²⁴ représente H, alkyle en C₁-C₂₀,
R²³ représente une simple liaison, O, NR⁷, ou un groupe de formule générale (IIa) selon la revendication 2,
sont utilisés.

5. Utilisation selon les revendications 1 à 3, dans laquelle le poids moléculaire des dérivés de benzotriazole de formule générale (I), (Ia) ou (Ib) est de 1 100 g/mol à 5 000 g/mol.

6. Utilisation de dérivés de benzotriazole selon les revendications 1 à 5 en tant qu'absorbeurs UV dans des plastiques ou des cires.

7. Utilisation de dérivés de benzotriazole selon les revendications 1 à 5 en tant qu'absorbeurs UV dans des corps moulés en plastique.

8. Utilisation de dérivés de benzotriazole selon les revendications 1 à 5 en tant qu'absorbeurs UV dans des textiles.

9. Utilisation de dérivés de benzotriazole selon les revendications 1 à 5 en tant qu'absorbeurs UV dans des préparations cosmétiques.

10. Utilisation de dérivés de benzotriazole selon les revendications 1 à 5 en tant qu'agents photoprotecteurs et stabilisateurs pour plastiques.

11. Procédé de stabilisation de plastiques contre l'effet de la lumière, éventuellement conjointement avec l'effet de l'oxygène sur le plastique, **caractérisé en ce que** des dérivés de benzotriazole selon les revendications 1 à 5 sont utilisés pour cela.

12. Matériaux organiques non vivants stabilisés contre l'effet de la lumière, de l'oxygène et de la chaleur, contenant 0,01 à 10 % en poids, par rapport à la quantité du matériau organique non vivant, de dérivés de benzotriazole selon les revendications 1 à 5.

13. Dérivés de benzotriazole selon la revendication 1, dans lesquels A correspond à un radical organique n-valent de formule (III) et n est un nombre entier dans la plage allant de 3 à 20.

14. Dérivés de benzotriazole selon la revendication 1, dans lesquels A correspond à un radical organique n-valent de formule (IIIa) et n est un nombre entier dans la plage allant de 5 à 20.

15. Dérivés de benzotriazole selon la revendication 1, dans lesquels A correspond à un radical organique n-valent de formule (IIIb) et n est un nombre entier dans la plage allant de 3 à 20.
